# EUROPEAN PATENT SPECIFICATION

(11) **EP 1 514 104 B1**
(45) Date of publication and mention of the grant of the patent: **03.10.2007**
(21) Application number: 02807433.4
(22) Date of filing: 20.05.2002
(51) Int. Cl.: G01N 33/53, C07K 17/00

(54) **PROTEINS WITH REPETITIVE BACTERIAL-IG-LIKE (BIG) DOMAINS PRESENT IN LEPTOSPIRA SPECIES**
IN LEPTOSPIRA-SPEZIES VORHANDENE PROTEINE MIT REPETITIVEN BAKTERIEN-IG-ÄHNLICHEN (BIG-) DOMÄNEN
PROTEINES AVEC DOMAINES DE REPETITION DE BACTERIE, TYPE IG (BIG), DANS L ESPECE LEPTOSPIRA

(30) Priority: 17.05.2002 US 147299
(43) Date of publication of application: 16.03.2005
(73) Proprietor: FUNDACAO OSWALDO CRUZ (FIOCRUZ), 21045-900 Rio de Janeiro (BR)
(72) Inventor: KO, Albert, I., C. de Pesquisas Gonçalo Moniz/Fio., CEP-40295-001 Salvador, BA (BR); GALVAO REIS, Mitermayer, Centro de Pesquisas, CEP-40295-001 Salvador, BA (BR); ROSA CRODA, Julio, H., Centro de Pesquisas, CEP-40295-001 Salvador, BA (BR); SIQUEIRA, Isadora, C., Centro de Pesquisas Goncalo, CEP-40295-001 Salvador, BA (BR); HAAKE, David, A., Los Angeles, CA (US); MATSUNAGA, James, Los Angeles, CA (US); RILEY, Lee, W., Berkeley, CA 94720 (US); BAROCCHI, Michele, Los Angeles (US); YOUNG, Tracy, A., Berkeley, CA 94720 (US)
(74) Representative: Stuttard, Garry Philip
(86) International application number: PCT/BR2002/000072
(87) International publication number: WO 2003/098214

(56) References cited:
- US-A- 5 091 301
- US-A- 5 989 547
- DATABASE EMBL 1 July 1997 (1997-07-01), JUSUF S.S.D.;: "Surface protein Lk90" XP002348172 Database accession no. O05734
- BUGHIO NASREEN I ET AL: "Use of recombinant flagellin protein as a tracer antigen in a fluorescence polarization assay for diagnosis of leptospirosis" CLINICAL AND DIAGNOSTIC LABORATORY IMMUNOLOGY, vol. 6, no. 4, July 1999 (1999-07), pages 599-605, XP002348169 ISSN: 1071-412X
- MATSUNAGA JAMES ET AL: "Pathogenic Leptospira species express surface-exposed proteins belonging to the bacterial immunoglobulin superfamily." MOLECULAR MICROBIOLOGY, vol. 49, no. 4, August 2003 (2003-08), pages 929-945, XP002348166 ISSN: 0950-382X
- PALANIAPPAN RAGHAVAN U M ET AL: "Cloning and molecular characterization of an immunogenic LigA protein of Leptospira interrogans." INFECTION AND IMMUNITY, vol. 70, no. 11, November 2002 (2002-11), pages 5924-5930, XP002348167 ISSN: 0019-9567
- REN SHUANG-XI ET AL: "Unique physiological and pathogenic features of Leptospira interrogans revealed by whole-genome sequencing." NATURE (LONDON), vol. 422, no. 6934, 24 April 2003 (2003-04-24), pages 888-893, XP002348168 ISSN: 0028-0836

### SEQUENCE LISTING

<110> FIOCRUZ
<120> Protein family with repetitive Bacterial-Ig-like (Big) domains present in Leptospira species
<130> GS/MPW/P207680
<140> EP02807433.4
   <141> 2002-05-20
<160> 10
<170> PatentIn version 3.3
<210> 1
   <211> 3672
   <212> DNA
   <213> Leptospira kirschneri
<400> 1
   atgaagagaa cattttgtat ttcgattctt ctttcgatgt tttttcaaag ttgtatgtct      60
   tggccacttt taaccagtct cgcgggttta gcagctggta aaaaaagtaa tgggctgccc     120
   tttttccacc ttctattaag taactctgat ccagttatta caaggatcga gctcagttat     180
   caaaattctt ccatcgcaaa aggtacaagt acaactctcg aagtcaccgc aatctttgat     240
   aacggaacaa atcagaatat tacggattcg acatctatcg tttccgatgc ccaatcaatc     300
   gttgacattc aaggtaacag agtcagagga atcgcttctg gttcttccat tataaaagct     360
   gaatacaacg ggatgtattc tgaacaaaaa attacggtta caccagccac gataaactca     420
   attcaagtta cgagtttaga tgacggtata ttacctaaag gtacaaatcg tcaatttgct     480
   gccatcggta tcttttcgga tggttctcat caagatattt ccaacgatcc attgatcgtt     540
   tggtcttcca gtaatataga tttagttcga gtagatgatt ccggtttggc ctcaggtatc     600
   aatttaggaa cggctcatat tcgtgcatcc tttcaatcaa aacaagcctc cgaagagata     660
   actgttggtg acgctgttct ttcttctatc caagtaactt ccaacagtcc aaatattcct     720
   ctcggaaaaa aacaaaaact cacagctact ggaatttatt cggataactc taacagggat     780
   atttcctctt ctgttatctg gaattcttct aattccacta tcgctaatat tcagaataac     840
   ggaatattag aaacagctga tactggaatt gttactgttt ctgcttctag aggtaatata     900
   aatggttcca taaaactaat cgtcactcct gctgccttag tttctatttc tgtttctcct     960
   acaaattctg cagtagcaaa aggtttacaa gaaaacttta aagctacagg gatctttaca    1020
   gataattcga actcagatat tacagatcaa gttacttggg attcttctaa tccggatatt    1080
   ctttccattt ccaatgcaag tgatagccac gggttagctt ccacactcaa ccaaggaaat    1140
   gttaaggtca ccgcttccat cggtggaata caaggatcca ctgattttaa agttacacaa    1200
   gaggtattaa cttccatcga agtttctcca gttttacctt caattgcaaa aggactaact    1260
   cagaaattta cggcgatcgg gatttttacg gataactcca aaaaagatat tacaaatcaa    1320
   gtcacttgga attcttcttc agcaatcgca agcgtgtcta acttagatga taataaaggt 1380
   ctgggaaaag ctcacgctgt tggagacacg actattaccg ctactttagg aaaagtttca 1440
   ggtaaaactt ggtttactgt agttcctgcg gttctcactt ctattcaaat caatcctgta 1500
   aatccttctc ttgcaaaagg gttaactcaa aaatttacgg ctactgggat ctactctgac 1560
   aactctaaca aggacattac ttcctccgtt acttggttct catccgattc ttcaatcgca 1620
   acaatttcaa acgccaaaaa aaatcaagga aactcttacg gagcagctac aggagcaacg 1680
   gatattaaag ccacattcgg aaaggtaagt agtccagttt ctacgttatc cgttactgct 1740
   gcaaaacttg ttgaaataca aatcacaccg gccgctgctt ccaaagcaaa gggaatttcc 1800
   gaaagattta aagcaaccgg tatttttaca gacaactcta attccgatat tacaaatcag 1860
   gtcacttgga gttcatctaa tacagatatt cttaccgttt ccaatacaaa cgccaaacgc 1920
   gggttaggtt ccactttaaa acaaggaact gttaaagtta tcgcttccat gggtggaatc 1980
   gaaagttctg tagattttac cgtcacacag gctaatttga cttcgatcga agtctctcca 2040
   actcgctctt cgattgcaaa aggactaact caaaaattta ccgctatagg tatttttacg 2100
   gatcattcta agaaggatat tacagagcaa gttacttgga agtcttcttc gaaagtatta 2160
   aatatgttga atgcatccgg tgaagaagga agaggtaagg caatttcagt cgggaaagcg 2220
   accattactg caaccttaga aaaactttcc gggaaagctg atattacagt tactcccgcg 2280
   gttcttactt caattcaaat cagtcctgtg aaaccttctc ttgtaaaagg gttaacagaa 2340
   aatttttctg ctacaggtat ctactctgat aattccagca aggacataac ttcctccgtt 2400
   acatggcatt cgttcaacaa ctctgttgca acgatctcga acacgaaaaa ttaccatgga 2460
   caagctcacg caaccggtac agggatagtg ggtattaaag cgacattggg aaatgtaagc 2520
   agcccagttt ccaaattatc cgttaccgca gcagaactgg ttgagattgt gttaaatcct 2580
   actttatctc acaaggccaa gggacttact gaaaatttta aagcgaccgg cgtatttacg 2640
   gacaattcga caaaagatat taccgaccag gttacttgga aatcttccaa tactgcctac 2700
   gcagaaattt caaacgcaac tggaagtaaa ggggttgtta atgcactctc gaagggaacg 2760
   agtcacattt ccgctacctt aggttcaatt tcaagtgcaa atgcgacatt ccaagttact 2820
   ccagcaaaaa tagcttcgat cgaaataaca ccaaataatt tcttcttgat caaaaaactt 2880
   agttatccat ttaaagcaat tggaatctat acggataata caaagacaga cattacaaaa 2940
   caagtttcct ggtcttcctc tgatccgaat gttgcatcga tcgataacac attttcattg 3000
   gctggctcag ctaccgcaat cgatgatgga aaaacgaaca tcactgcaac gttatccgac 3060
   tctatgtccg cttccactac tttgtatgtc acttctgcta cgcttgttga catagaagta 3120
   aaacctagta tcttcgttct gagtgaaggt cttacactac aactgaccgc taccggcatc 3180
   tattcggatt actctaccta tgatttgact caggttgtaa cgtggacttc cagcgaacca 3240
   tccaacattt cgatcgaaaa tacagccggt aaaaaaggta aagtaacggc tcttgcattt 3300
   ggagcttcag aatttacggc aacctacgat tctattgaaa gtaatcgagc ttggatattt 3360
   gtcaatgacg agaaatttgt aaacataacc attagttctt ctcaagtttt gacagacaag 3420
   ggcttgactc aacaattcaa agcaatcgga actttcgaaa aaggtagcga acttgacctt 3480
   acggatcttg taacctggaa gtcctctgat tctaaggtag cttctatcgg taactctaat 3540
   gatgacagag gtttaataac accgctttct gtaggttcct ctaaaatttc tgcgacttac 3600
   aattctatcc atagtaactc tattgatttt gaagtaactc cagaaatatt agcctctatt 3660
   aaaacgaagc cg 3672
<210> 2
   <211> 1224
   <212> PRT
   <213> Leptospira kirschneri
<400> 2
   Met Lys Arg Thr Phe Cys Ile Ser Ile Leu Leu Ser Met Phe Phe Gln
   1               5                   10                  15
   Ser Cys Met Ser Trp Pro Leu Leu Thr Ser Leu Ala Gly Leu Ala Ala
               20                  25                  30
   Gly Lys Lys Ser Asn Gly Leu Pro Phe Phe His Leu Leu Leu Ser Asn
           35                  40                  45
   Ser Asp Pro Val Ile Thr Arg Ile Glu Leu Ser Tyr Gln Asn Ser Ser
       50                  55                  60
   Ile Ala Lys Gly Thr Ser Thr Thr Leu Glu Val Thr Ala Ile Phe Asp
   65                  70                  75                      80
   Asn Gly Thr Asn Gln Asn Ile Thr Asp Ser Thr Ser Ile Val Ser Asp
                   85                  90                  95
   Ala Gln Ser Ile Val Asp Ile Gln Gly Asn Arg Val Arg Gly Ile Ala
               100                 105                 110
   Ser Gly Ser Ser Ile Ile Lys Ala Glu Tyr Asn Gly Met Tyr Ser Glu
           115                 120                 125
   Gln Lys Ile Thr Val Thr Pro Ala Thr Ile Asn Ser Ile Gln Val Thr
       130                 135                 140
   Ser Leu Asp Asp Gly Ile Leu Pro Lys Gly Thr Asn Arg Gln Phe Ala
   145                 150                 155                     160
   Ala Ile Gly Ile Phe Ser Asp Gly Ser His Gln Asp Ile Ser Asn Asp
                   165                 170                 175
   Pro Leu Ile Val Trp Ser Ser Ser Asn Ile Asp Leu Val Arg Val Asp
   180 185 190
   Asp Ser Gly Leu Ala Ser Gly Ile Asn Leu Gly Thr Ala His Ile Arg
   195 200 205
   Ala Ser Phe Gln Ser Lys Gln Ala Ser Glu Glu Ile Thr Val Gly Asp
   210 215 220
   Ala Val Leu Ser Ser Ile Gln Val Thr Ser Asn Ser Pro Asn Ile Pro
   225 230 235 240
   Leu Gly Lys Lys Gln Lys Leu Thr Ala Thr Gly Ile Tyr Ser Asp Asn
   245 250 255
   Ser Asn Arg Asp Ile Ser Ser Ser Val Ile Trp Asn Ser Ser Asn Ser
   260 265 270
   Thr Ile Ala Asn Ile Gln Asn Asn Gly Ile Leu Glu Thr Ala Asp Thr
   275 280 285
   Gly Ile Val Thr Val Ser Ala Ser Arg Gly Asn Ile Asn Gly Ser Ile
   290 295 300
   Lys Leu Ile Val Thr Pro Ala Ala Leu Val Ser Ile Ser Val Ser Pro
   305 310 315 320
   Thr Asn Ser Ala Val Ala Lys Gly Leu Gln Glu Asn Phe Lys Ala Thr
   325 330 335
   Gly Ile Phe Thr Asp Asn Ser Asn Ser Asp Ile Thr Asp Gln Val Thr
   340 345 350
   Trp Asp Ser Ser Asn Pro Asp Ile Leu Ser Ile Ser Asn Ala Ser Asp
   355 360 365
   Ser His Gly Leu Ala Ser Thr Leu Asn Gln Gly Asn Val Lys Val Thr
   370 375 380
   Ala Ser Ile Gly Gly Ile Gln Gly Ser Thr Asp Phe Lys Val Thr Gln
   385 390 395 400
   Glu Val Leu Thr Ser Ile Glu Val Ser Pro Val Leu Pro Ser Ile Ala
   405 410 415
   Lys Gly Leu Thr Gln Lys Phe Thr Ala Ile Gly Ile Phe Thr Asp Asn
   420 425 430
   Ser Lys Lys Asp Ile Thr Asn Gln Val Thr Trp Asn Ser Ser Ser Ala
   435 440 445
   Ile Ala Ser Val Ser Asn Leu Asp Asp Asn Lys Gly Leu Gly Lys Ala
       450                 455                 460
   His Ala Val Gly Asp Thr Thr Ile Thr Ala Thr Leu Gly Lys Val Ser
   465                 470                 475                 480
   Gly Lys Thr Trp Phe Thr Val Val Pro Ala Val Leu Thr Ser Ile Gln
                   485                 490                 495
   Ile Asn Pro Val Asn Pro Ser Leu Ala Lys Gly Leu Thr Gln Lys Phe
               500                 505                 510
   Thr Ala Thr Gly Ile Tyr Ser Asp Asn Ser Asn Lys Asp Ile Thr Ser
           515                 520                 525
   Ser Val Thr Trp Phe Ser Ser Asp Ser Ser Ile Ala Thr Ile Ser Asn
       530                 535                 540
   Ala Lys Lys Asn Gln Gly Asn Ser Tyr Gly Ala Ala Thr Gly Ala Thr
   545                 550                 555                 560
   Asp Ile Lys Ala Thr Phe Gly Lys Val Ser Ser Pro Val Ser Thr Leu
                   565                 570                 575
   Ser Val Thr Ala Ala Lys Leu Val Glu Ile Gln Ile Thr Pro Ala Ala
               580                 585                 590
   Ala Ser Lys Ala Lys Gly Ile Ser Glu Arg Phe Lys Ala Thr Gly Ile
           595                 600                 605
   Phe Thr Asp Asn Ser Asn Ser Asp Ile Thr Asn Gln Val Thr Trp Ser
       610                 615                 620
   Ser Ser Asn Thr Asp Ile Leu Thr Val Ser Asn Thr Asn Ala Lys Arg
   625                 630                 635                 640
   Gly Leu Gly Ser Thr Leu Lys Gln Gly Thr Val Lys Val Ile Ala Ser
                   645                 650                 655
   Met Gly Gly Ile Glu Ser Ser Val Asp Phe Thr Val Thr Gln Ala Asn
               660                 665                 670
   Leu Thr Ser Ile Glu Val Ser Pro Thr Arg Ser Ser Ile Ala Lys Gly
           675                 680                 685
   Leu Thr Gln Lys Phe Thr Ala Ile Gly Ile Phe Thr Asp His Ser Lys
       690                 695                 700
   Lys Asp Ile Thr Glu Gln Val Thr Trp Lys Ser Ser Ser Lys Val Leu
   705 710 715 720
   Asn Met Leu Asn Ala Ser Gly Glu Glu Gly Arg Gly Lys Ala Ile Ser
   725 730 735
   Val Gly Lys Ala Thr Ile Thr Ala Thr Leu Glu Lys Leu Ser Gly Lys
   740 745 750
   Ala Asp Ile Thr Val Thr Pro Ala Val Leu Thr Ser Ile Gln Ile Ser
   755 760 765
   Pro Val Lys Pro Ser Leu Val Lys Gly Leu Thr Glu Asn Phe Ser Ala
   770 775 780
   Thr Gly Ile Tyr Ser Asp Asn Ser Ser Lys Asp Ile Thr Ser Ser Val
   785 790 795 800
   Thr Trp His Ser Phe Asn Asn Ser Val Ala Thr Ile Ser Asn Thr Lys
   805 810 815
   Asn Tyr His Gly Gln Ala His Ala Thr Gly Thr Gly Ile Val Gly Ile
   820 825 830
   Lys Ala Thr Leu Gly Asn Val Ser Ser Pro Val Ser Lys Leu Ser Val
   835 840 845
   Thr Ala Ala Glu Leu Val Glu Ile Val Leu Asn Pro Thr Leu Ser His
   850 855 860
   Lys Ala Lys Gly Leu Thr Glu Asn Phe Lys Ala Thr Gly Val Phe Thr
   865 870 875 880
   Asp Asn Ser Thr Lys Asp Ile Thr Asp Gln Val Thr Trp Lys Ser Ser
   885 890 895
   Asn Thr Ala Tyr Ala Glu Ile Ser Asn Ala Thr Gly Ser Lys Gly Val
   900 905 910
   Val Asn Ala Leu Ser Lys Gly Thr Ser His Ile Ser Ala Thr Leu Gly
   915 920 925
   Ser Ile Ser Ser Ala Asn Ala Thr Phe Gln Val Thr Pro Ala Lys Ile
   930 935 940
   Ala Ser Ile Glu Ile Thr Pro Asn Asn Phe Phe Leu Ile Lys Lys Leu
   945 950 955 960
   Ser Tyr Pro Phe Lys Ala Ile Gly Ile Tyr Thr Asp Asn Thr Lys Thr
   965 970 975
   Asp Ile Thr Lys Gln Val Ser Trp Ser Ser Ser Asp Pro Asn Val Ala
               980                 985                 990
   Ser Ile Asp Asn Thr Phe Ser Leu Ala Gly Ser Ala Thr Ala Ile Asp
           995                 1000                1005
   Asp Gly Lys Thr Asn Ile Thr Ala Thr Leu Ser Asp Ser Met Ser
       1010                1015                1020
   Ala Ser Thr Thr Leu Tyr Val Thr Ser Ala Thr Leu Val Asp Ile
       1025                1030                1035
   Glu Val Lys Pro Ser Ile Phe Val Leu Ser Glu Gly Leu Thr Leu
       1040                1045                1050
   Gln Leu Thr Ala Thr Gly Ile Tyr Ser Asp Tyr Ser Thr Tyr Asp
       1055                1060                1065
   Leu Thr Gln Val Val Thr Trp Thr Ser Ser Glu Pro Ser Asn Ile
       1070                1075                1080
   Ser Ile Glu Asn Thr Ala Gly Lys Lys Gly Lys Val Thr Ala Leu
       1085                1090                1095
   Ala Phe Gly Ala Ser Glu Phe Thr Ala Thr Tyr Asp Ser Ile Glu
       1100                1105                1110
   Ser Asn Arg Ala Trp Ile Phe Val Asn Asp Glu Lys Phe Val Asn
       1115                1120                1125
   Ile Thr Ile Ser Ser Ser Gln Val Leu Thr Asp Lys Gly Leu Thr
       1130                1135                1140
   Gln Gln Phe Lys Ala Ile Gly Thr Phe Glu Lys Gly Ser Glu Leu
       1145                1150                1155
   Asp Leu Thr Asp Leu Val Thr Trp Lys Ser Ser Asp Ser Lys Val
       1160                1165                1170
   Ala Ser Ile Gly Asn Ser Asn Asp Asp Arg Gly Leu Ile Thr Pro
       1175                1180                1185
   Leu Ser Val Gly Ser Ser Lys Ile Ser Ala Thr Tyr Asn Ser Ile
       1190                1195                1200
   His Ser Asn Ser Ile Asp Phe Glu Val Thr Pro Glu Ile Leu Ala
       1205                1210                1215
   Ser Ile Lys Thr Lys Pro
   1220
<210> 3
   <211> 5863
   <212> DNA
   <213> Leptospira kirschneri
<400> 3
   atgcctaaac atatcaacaa actcagagat aaaaaaacgt ggccttttct tcagtttatt     60
   tttattcttt ttctaacatt cagcctattt tttttggaaa gttgcgcggc ttggccaatt    120
   ttttcaggca cacctggttt attagcaggt aaaaaaagcg gagcaaacaa ttcactttgg    180
   atgctttttt taggaataga taatccgctc gaatcggagc catccgaagc agagttagat    240
   cggatcgaaa tttccgtacc gaactcaaat ttagctcgag gtactacttt acatctaaac    300
   gccacagcca tctataaaga caatactcac cgagatattt cttcggaagg atcctggtcc    360
   tctacggatt cgagcattct caagctatta acacaatctc aattcaaagg aatgaatcta    420
   ggttctggaa acgttaatgt atcctttcaa ggaaaaaacg caactacaac gttaaccgtt    480
   acatccgctg ttttgtccga tctgaccgta acttgtgtga accaaggtag tccattacct    540
   gttggaatcg atcgtcaatg taaattagaa ggaatttttt cggacggtag tactcaggtt    600
   ttaacttccg atccaagcgc gtcctggaac gtaacccaat cttctattgc aggtgtaaac    660
   accacaggtt tagtttccgg actttctcca ggtaacactt ttattaccac ttcttatgga    720
   agtaaaacct ccagtttgaa tgtgaccgta agtgcggcaa cccttagctc gatctcagtg    780
   actcctgcca actcaagtta tcctcttggc aaggtccaac agtacacagc aatcggaacc    840
   tacagcaatc agtccactca agatttaaca aatcaggttt cctgggcttc tttaaatact    900
   tccgttgcta cgatcgataa ttctacatcc gccaaaggta tgcttactac tcaatcaacc    960
   ggttcagcaa acatcacggc aacgttaggc ggaattaccg gacagactac tagtaaacgt   1020
   cacttccgca gttcttacta gtattacgat cactcctgca aatccaagcg tagccaatgg   1080
   aaggacatta tatcttaccg ccaccggagt tttttcggat ggtacagttt ccgacattac   1140
   caaccaagta acttggtcca gttccttaac aagtgtagct accgcagata actcaggcgg   1200
   tttatccgga agaatttccg gagtcggagt tggtagtacg aatatcaccg ccgccatcgg   1260
   tggagtagat attacggttt ctttaaatgt taccaacgcc actttagaat cgattcaagt   1320
   ggtttccgat tcccattcga tagctcgagg tacgtctacg tttgtacaag cgataggagt   1380
   ctactcggac ggttcttctc aaaacataag tgatcaagtt gcctggaaca gctctaattc   1440
   ttcaatatta caaatatcta atttaaatgc agttcccaaa agagaaatac aatctccttc   1500
   ttccggaggc ctaggtacag caaggatcac cgcaacttta gaagcaatct cctcatatac   1560
   cgacatctcg gtcaatgcag caactttagt ttctatcgaa gtgtcaccca caaatccttc   1620
   ggtatcttca ggacttaccg ttccttttac ggcgaccgga gtttatacgg atggaagtaa   1680
   tcaaaatctg acttctcaag taacttggaa ttcctccaac acgaacagag ctacaatcag 1740
   caacgcaaac ggaactcaag gaattgcctt gggctcttct gtcggaacta cgaacatatc 1800
   agcaacgtta ggtgcggtta cttcttccgc taccactctt acggtcacaa acgcggtttt 1860
   aaattcgatc acgattactc cgtctcttcc ttccgtagca gtaggaagaa gtctgaacct 1920
   tactgcaacc ggaacttatt ctgacggaag taaccaagat ttaactacct ccgtcgcttg 1980
   gacgagtacg gattcttcca tcgtttccgt agacaacgcc tcaggtagac aggggcagac 2040
   gacaggtgtt gcacaaggta acactcagat cagtgccaca ttaggcggaa cttcttctgc 2100
   tatcaatttt acggtaagtg cagcggtttt agattcaatt caagtaactc tggaagattc 2160
   tccgattgca aaaggaactt ctacaagagc aatcgcgacg ggtgtttttt cagacggaag 2220
   caatttgaat attagtgatc aagttatttg ggatagttca caaacaaacg tgatccagct 2280
   aggagtttta gaaaccggtc ctaaaaagaa actgatgaat tctcccgcaa atggaaacag 2340
   taccactgga acctcaagga tcactgcaac gttaggaggt gtgagcggat acgccgatct 2400
   tacagtaatc gctccaagtt taaccagcat tcaaatcgat cctacacatc cgagcgttgc 2460
   caacggtctg actcaaaatt ttactgcaac cggagtttac tcagatggta gcaatcagaa 2520
   tctaaccgat tccgttactt gggcgtcttc caatcctgct gttgccacga tcagcaacgc 2580
   ttccggaacc aacggtaaag ctactactct tcaaactgga tccaccaata tcagcgcgag 2640
   tctgggcgcc actacttctg atccaagtgt attaacggtt acaaacgcaa ccttaacaag 2700
   tatcacgatc gctcccacct cttccttcaa catcgcaaaa ggattaaatc aagactttgt 2760
   agcgaccggt tattatacag atggttcttc tagagacctg accactcaag tcacttggaa 2820
   ttcttccaat acttctaccg ctacgatcag caatgcaaac ggaactcaag gaagaatggc 2880
   cgcggtcgat actggttcta caaatatctc cgcgtcttta ggaggaacgt atagtcagac 2940
   cacaaacgta accgttacat ctgcggttct gaattcgatc caggtttctc cagcggacat 3000
   tagtgtagcc aaaggaaaca ccaaggccta caccgcgatc ggagtatatt cagattttag 3060
   cacgttagac gttacttctc aggttacctg gacttcttcc agcgtttcga tcgctacgat 3120
   cagcaatgca agcggacacg aaggtttagc tacggctgta ggcacgggaa cttccacaat 3180
   taccgcaact cttggaggaa tttctaattc tacgagtttg acggttacgg ccgccgtatt 3240
   ggtttctctt tcggtaggtc ctaccaatag ttttgtttat atgacacaaa ccaaaaattt 3300
   tatggctact ggaacgtatt ctgacggaac gatgcaggat cttacaactc aagtcacctg 3360
   gacttcttcc gatacaacct tgggaacaat cagcaacgcc ttcggaatag aaggtagggc 3420
   tacaggaatt gctgccggtg ccataacgat cactgcgact ttgggaagta tcagcggaaa 3480
   cacttctttg actataatct ttttagatac gatagcacct gcgatcacaa acgtagtcgc 3540
   cttaactcct actactttaa gaattacata ttccgaaaac gtaaacgaaa cccaggcaaa 3600
   aaccgcggcc aattacaaac tggctcttac ttcttccgta accggaagtt gttcagataa 3660
   cagcaacttt acttctacct cttctgtgat tactgtttcc tcagtgagtg gaagcggatc 3720
   tgtgtttgtt ctaactctag gttcttcaca aacgtctaac gcaccttata cgattttagt 3780
   gaataaatcg ggaatacaag atctttctac aaccccaaac aatttgggtt gtgcaaacta 3840
   cggagacttc ttaggacagg aacaaatcaa aatcgtatcc gcctcctgtg caaattccaa 3900
   ttccgtgatt ttgaatttct ctaaggctcc taaatctgga aacaatgtcg ccggttccgc 3960
   agaatgtacc ggttctgcag aatgttctaa tcgttacaaa atttccggag caagcgatct 4020
   tggaacaatt aacagcgtaa aggtgttaga tggaattatt tgtaacggag caactgcaga 4080
   ttccgcaaaa gtatgcgtaa ttcataattt agtacaaacc ggagcacaat atacaatcat 4140
   cactgcggat tccgtagacg gagacggatt tgacaactca agctggggat caatccgaaa 4200
   ttctttggat acagagaatc ttcaatcttc tcccagagac agggcttcct ttttaggatg 4260
   tggaacgtct ccggtcaact ttgcagacgg accgatttcc atcgatccaa actcatccac 4320
   gttcggttat ctaatcgatt ttaactctaa gatctattca ggaccaaaca attccgggaa 4380
   cggagcgctt cgatttgcct atgatggaag tgttccagaa tcagttcaat tctcctttga 4440
   aaaagacaca accgttcaag acggtgacgc gactaacgta agttcaaact cagcttcttc 4500
   cagagagaat tcgatctcgg ttccgcctta cgttacatta ggacactccg gatgtactac 4560
   aaacaacgga actctttctc taggatgtgg tccggataac gaaaacggaa gaggagtatt 4620
   cgctactgga attctttcca gcgtctccta tctatttgtt gcagctgcaa aaaccgtagc 4680
   ggacggcctg ggacaatact tatttgatta tctgtattac tccgcagaca cttctactaa 4740
   tacaagtttc aaatatatag atctaggatc gatcaccgga actttaaccg ccggaacttc 4800
   ttcgcttact gtactcaata atagagtgtt tgcaggtttt gcaaagtcaa gcaacgacgg 4860
   aatcggattg ttcggaggac ttaatgcacc cgattttgga tttgtaacgt ttaactcagc 4920
   ggactcagga actggatttt gtactccagg ctccaactgc gacgcgtttg acggaaccaa 4980
   aggaaaaaga atccggatcg atttccttcc ttacttcgga ggaccgtcca ccggtttatt 5040
   aggaattaat aataatgcac atccaaactg ggcgtattat atcggagtcg attccatgtt 5100
   cgtatttaaa aatcgtatct atgccgcaaa cggaggatta cacgcggtag gacataacgg 5160
   ttccataata cgttctacaa ctgcagatcc aaccgcggct tgtaccggac cggactcttg 5220
   ttctaactgg gtggaaattg gacctagaac caacacgaaa tggcacaaca gtcccacaaa 5280
   caactggttc tctttagagt taaatcaatt ttacaatctg attccgggag ataaggcgtt 5340
   tgcacaattt gccgagttca acaataacct ttatgtaact agaaccattt gtattcaaag 5400
   ttctcaagcg actggaatca gaaccaatcc aggaaccgta acaggatgta cagacggaac 5460
   aactacaaat cgaagggcac aactttggaa atgtgatcct acaatttcag gaaacacgag 5520
   cgaatgtgat gcagcggatt ggtcggtcgt aggcgacgac ggaaccggaa tcacaaacat 5580
   gggagattct acaaaccgaa cgatcaccat ggtgatgaaa aacggatcct atctttacat 5640
   aggatatgat aatccaaacg gaatcagaat ttatagaacc aacgtagcca acccgggatc 5700
   atcctctgcg tcttggagtc aaatcgccgg gaacggtctc acagatgcga ctaacgttca 5760
   acaaatttac tcggccgtat ccgtaccttc cggaagtatc aattatatct acgtaagcgc 5820
   tggaaaaagt aacgtttctg ttcggacgta tcgtcaacaa aat 5863
<210> 4
   <211> 1954
   <212> PRT
   <213> Leptospira kirschneri
<400> 4
   Met Pro Lys His Ile Asn Lys Leu Arg Asp Lys Lys Thr Trp Pro Phe
   1               5                   10                  15
   Leu Gln Phe Ile Phe Ile Leu Phe Leu Thr Phe Ser Leu Phe Phe Leu
               20                  25                  30
   Glu Ser Cys Ala Ala Trp Pro Ile Phe Ser Gly Thr Pro Gly Leu Leu
           35                  40                  45
   Ala Gly Lys Lys Ser Gly Ala Asn Asn Ser Leu Trp Met Leu Phe Leu
       50                  55                  60
   Gly Ile Asp Asn Pro Leu Glu Ser Glu Pro Ser Glu Ala Glu Leu Asp
   65                  70                  75                  80
   Arg Ile Glu Ile Ser Val Pro Asn Ser Asn Leu Ala Arg Gly Thr Thr
                   85                  90                  95
   Leu His Leu Asn Ala Thr Ala Ile Tyr Lys Asp Asn Thr His Arg Asp
               100                 105                 110
   Ile Ser Ser Glu Gly Ser Trp Ser Ser Thr Asp Ser Ser Ile Leu Lys
           115                 120                 125
   Leu Leu Thr Gln Ser Gln Phe Lys Gly Met Asn Leu Gly Ser Gly Asn
       130                 135                 140
   Val Asn Val Ser Phe Gln Gly Lys Asn Ala Thr Thr Thr Leu Thr Val
   145                 150                 155                 160
   Thr Ser Ala Val Leu Ser Asp Leu Thr Val Thr Cys Val Asn Gln Gly
                   165                 170                 175
   Ser Pro Leu Pro Val Gly Ile Asp Arg Gln Cys Lys Leu Glu Gly Ile
               180                 185                 190
   Phe Ser Asp Gly Ser Thr Gln Val Leu Thr Ser Asp Pro Ser Ala Ser
           195                 200                 205
   Trp Asn Val Thr Gln Ser Ser Ile Ala Gly Val Asn Thr Thr Gly Leu
       210                 215                 220
   Val Ser Gly Leu Ser Pro Gly Asn Thr Phe Ile Thr Thr Ser Tyr Gly
   225                 230                 235                 240
   Ser Lys Thr Ser Ser Leu Asn Val Thr Val Ser Ala Ala Thr Leu Ser
                   245                 250                 255
   Ser Ile Ser Val Thr Pro Ala Asn Ser Ser Tyr Pro Leu Gly Lys Val
               260                 265                 270
   Gln Gln Tyr Thr Ala Ile Gly Thr Tyr Ser Asn Gln Ser Thr Gln Asp
           275                 280                 285
   Leu Thr Asn Gln Val Ser Trp Ala Ser Leu Asn Thr Ser Val Ala Thr
       290                 295                 300
   Ile Asp Asn Ser Thr Ser Ala Lys Gly Met Leu Thr Thr Gln Ser Thr
   305                 310                 315                 320
   Gly Ser Ala Asn Ile Thr Ala Thr Leu Gly Gly Ile Thr Gly Gln Thr
                   325                 330                 335
   Thr Val Asn Val Thr Ser Ala Val Leu Thr Ser Ile Thr Ile Thr Pro
               340                 345                 350
   Ala Asn Pro Ser Val Ala Asn Gly Arg Thr Leu Tyr Leu Thr Ala Thr
           355                 360                 365
   Gly Val Phe Ser Asp Gly Thr Val Ser Asp Ile Thr Asn Gln Val Thr
       370                 375                 380
   Trp Ser Ser Ser Leu Thr Ser Val Ala Thr Ala Asp Asn Ser Gly Gly
   385                 390                 395                 400
   Leu Ser Gly Arg Ile Ser Gly Val Gly Val Gly Ser Thr Asn Ile Thr
                   405                 410                 415
   Ala Ala Ile Gly Gly Val Asp Ile Thr Val Ser Leu Asn Val Thr Asn
               420                 425                 430
   Ala Thr Leu Glu Ser Ile Gln Val Val Ser Asp Ser His Ser Ile Ala
           435                 440                 445
   Arg Gly Thr Ser Thr Phe Val Gln Ala Ile Gly Val Tyr Ser Asp Gly
       450                 455                 460
   Ser Ser Gln Asn Ile Ser Asp Gln Val Ala Trp Asn Ser Ser Asn Ser
   465 470 475 480
   Ser Ile Leu Gln Ile Ser Asn Leu Asn Ala Val Pro Lys Arg Glu Ile
   485 490 495
   Gln Ser Pro Ser Ser Gly Gly Leu Gly Thr Ala Arg Ile Thr Ala Thr
   500 505 510
   Leu Glu Ala Ile Ser Ser Tyr Thr Asp Ile Ser Val Asn Ala Ala Thr
   515 520 525
   Leu Val Ser Ile Glu Val Ser Pro Thr Asn Pro Ser Val Ser Ser Gly
   530 535 540
   Leu Thr Val Pro Phe Thr Ala Thr Gly Val Tyr Thr Asp Gly Ser Asn
   545 550 555 560
   Gln Asn Leu Thr Ser Gln Val Thr Trp Asn Ser Ser Asn Thr Asn Arg
   565 570 575
   Ala Thr Ile Ser Asn Ala Asn Gly Thr Gln Gly Ile Ala Leu Gly Ser
   580 585 590
   Ser Val Gly Thr Thr Asn Ile Ser Ala Thr Leu Gly Ala Val Thr Ser
   595 600 605
   Ser Ala Thr Thr Leu Thr Val Thr Asn Ala Val Leu Asn Ser Ile Thr
   610 615 620
   Ile Thr Pro Ser Leu Pro Ser Val Ala Val Gly Arg Ser Leu Asn Leu
   625 630 635 640
   Thr Ala Thr Gly Thr Tyr Ser Asp Gly Ser Asn Gln Asp Leu Thr Thr
   645 650 655
   Ser Val Ala Trp Thr Ser Thr Asp Ser Ser Ile Val Ser Val Asp Asn
   660 665 670
   Ala Ser Gly Arg Gln Gly Gln Thr Thr Gly Val Ala Gln Gly Asn Thr
   675 680 685
   Gln Ile Ser Ala Thr Leu Gly Gly Thr Ser Ser Ala Ile Asn Phe Thr
   690 695 700
   Val Ser Ala Ala Val Leu Asp Ser Ile Gln Val Thr Leu Glu Asp Ser
   705 710 715 720
   Pro Ile Ala Lys Gly Thr Ser Thr Arg Ala Ile Ala Thr Gly Val Phe
   725 730 735
   Ser Asp Gly Ser Asn Leu Asn Ile Ser Asp Gln Val Ile Trp Asp Ser
   740 745 750
   Ser Gln Thr Asn Val Ile Gln Leu Gly Val Leu Glu Thr Gly Pro Lys
   755 760 765
   Lys Lys Leu Met Asn Ser Pro Ala Asn Gly Asn Ser Thr Thr Gly Thr
   770 775 780
   Ser Arg Ile Thr Ala Thr Leu Gly Gly Val Ser Gly Tyr Ala Asp Leu
   785 790 795 800
   Thr Val Ile Ala Pro Ser Leu Thr Ser Ile Gln Ile Asp Pro Thr His
   805 810 815
   Pro Ser Val Ala Asn Gly Leu Thr Gln Asn Phe Thr Ala Thr Gly Val
   820 825 830
   Tyr Ser Asp Gly Ser Asn Gln Asn Leu Thr Asp Ser Val Thr Trp Ala
   835 840 845
   Ser Ser Asn Pro Ala Val Ala Thr Ile Ser Asn Ala Ser Gly Thr Asn
   850 855 860
   Gly Lys Ala Thr Thr Leu Gln Thr Gly Ser Thr Asn Ile Ser Ala Ser
   865 870 875 880
   Leu Gly Ala Thr Thr Ser Asp Pro Ser Val Leu Thr Val Thr Asn Ala
   885 890 895
   Thr Leu Thr Ser Ile Thr Ile Ala Pro Thr Ser Ser Phe Asn Ile Ala
   900 905 910
   Lys Gly Leu Asn Gln Asp Phe Val Ala Thr Gly Tyr Tyr Thr Asp Gly
   915 920 925
   Ser Ser Arg Asp Leu Thr Thr Gln Val Thr Trp Asn Ser Ser Asn Thr
   930 935 940
   Ser Thr Ala Thr Ile Ser Asn Ala Asn Gly Thr Gln Gly Arg Met Ala
   945 950 955 960
   Ala Val Asp Thr Gly Ser Thr Asn Ile Ser Ala Ser Leu Gly Gly Thr
   965 970 975
   Tyr Ser Gln Thr Thr Asn Val Thr Val Thr Ser Ala Val Leu Asn Ser
   980 985 990
   Ile Gln Val Ser Pro Ala Asp Ile Ser Val Ala Lys Gly Asn Thr Lys
   995 1000 1005
   Ala Tyr Thr Ala Ile Gly Val Tyr Ser Asp Phe Ser Thr Leu Asp
   1010 1015 1020
   Val Thr Ser Gln Val Thr Trp Thr Ser Ser Ser Val Ser Ile Ala
   1025 1030 1035
   Thr Ile Ser Asn Ala Ser Gly His Glu Gly Leu Ala Thr Ala Val
   1040 1045 1050
   Gly Thr Gly Thr Ser Thr Ile Thr Ala Thr Leu Gly Gly Ile Ser
   1055 1060 1065
   Asn Ser Thr Ser Leu Thr Val Thr Ala Ala Val Leu Val Ser Leu
   1070 1075 1080
   Ser Val Gly Pro Thr Asn Ser Phe Val Tyr Met Thr Gln Thr Lys
   1085 1090 1095
   Asn Phe Met Ala Thr Gly Thr Tyr Ser Asp Gly Thr Met Gln Asp
   1100 1105 1110
   Leu Thr Thr Gln Val Thr Trp Thr Ser Ser Asp Thr Thr Leu Gly
   1115 1120 1125
   Thr Ile Ser Asn Ala Phe Gly Ile Glu Gly Arg Ala Thr Gly Ile
   1130 1135 1140
   Ala Ala Gly Ala Ile Thr Ile Thr Ala Thr Leu Gly Ser Ile Ser
   1145 1150 1155
   Gly Asn Thr Ser Leu Thr Ile Ile Phe Leu Asp Thr Ile Ala Pro
   1160 1165 1170
   Ala Ile Thr Asn Val Val Ala Leu Thr Pro Thr Thr Leu Arg Ile
   1175 1180 1185
   Thr Tyr Ser Glu Asn Val Asn Glu Thr Gln Ala Lys Thr Ala Ala
   1190 1195 1200
   Asn Tyr Lys Leu Ala Leu Thr Ser Ser Val Thr Gly Ser Cys Ser
   1205 1210 1215
   Asp Asn Ser Asn Phe Thr Ser Thr Ser Ser Val Ile Thr Val Ser
   1220 1225 1230
   Ser Val Ser Gly Ser Gly Ser Val Phe Val Leu Thr Leu Gly Ser
   1235 1240 1245
   Ser Gln Thr Ser Asn Ala Pro Tyr Thr Ile Leu Val Asn Lys Ser
   1250 1255 1260
   Gly Ile Gln Asp Leu Ser Thr Thr Pro Asn Asn Leu Gly Cys Ala
   1265 1270 1275
   Asn Tyr Gly Asp Phe Leu Gly Gln Glu Gln Ile Lys Ile Val Ser
   1280 1285 1290
   Ala Ser Cys Ala Asn Ser Asn Ser Val Ile Leu Asn Phe Ser Lys
   1295 1300 1305
   Ala Pro Lys Ser Gly Asn Asn Val Ala Gly Ser Ala Glu Cys Thr
   1310 1315 1320
   Gly Ser Ala Glu Cys Ser Asn Arg Tyr Lys Ile Ser Gly Ala Ser
   1325 1330 1335
   Asp Leu Gly Thr Ile Asn Ser Val Lys Val Leu Asp Gly Ile Ile
   1340 1345 1350
   Cys Asn Gly Ala Thr Ala Asp Ser Ala Lys Val Cys Val Ile His
   1355 1360 1365
   Asn Leu Val Gln Thr Gly Ala Gln Tyr Thr Ile Ile Thr Ala Asp
   1370 1375 1380
   Ser Val Asp Gly Asp Gly Phe Asp Asn Ser Ser Trp Gly Ser Ile
   1385 1390 1395
   Arg Asn Ser Leu Asp Thr Glu Asn Leu Gln Ser Ser Pro Arg Asp
   1400 1405 1410
   Arg Ala Ser Phe Leu Gly Cys Gly Thr Ser Pro Val Asn Phe Ala
   1415 1420 1425
   Asp Gly Pro Ile Ser Ile Asp Pro Asn Ser Ser Thr Phe Gly Tyr
   1430 1435 1440
   Leu Ile Asp Phe Asn Ser Lys Ile Tyr Ser Gly Pro Asn Asn Ser
   1445 1450 1455
   Gly Asn Gly Ala Leu Arg Phe Ala Tyr Asp Gly Ser Val Pro Glu
   1460 1465 1470
   Ser Val Gln Phe Ser Phe Glu Lys Asp Thr Thr Val Gln Asp Gly
   1475 1480 1485
   Asp Ala Thr Asn Val Ser Ser Asn Ser Ala Ser Ser Arg Glu Asn
       1490                1495                1500
   Ser Ile Ser Val Pro Pro Tyr Val Thr Leu Gly His Ser Gly Cys
       1505                1510                1515
   Thr Thr Asn Asn Gly Thr Leu Ser Leu Gly Cys Gly Pro Asp Asn
       1520                1525                1530
   Glu Asn Gly Arg Gly Val Phe Ala Thr Gly Ile Leu Ser Ser Val
       1535                1540                1545
   Ser Tyr Leu Phe Val Ala Ala Ala Lys Thr Val Ala Asp Gly Leu
       1550                1555                1560
   Gly Gln Tyr Leu Phe Asp Tyr Leu Tyr Tyr Ser Ala Asp Thr Ser
       1565                1570                1575
   Thr Asn Thr Ser Phe Lys Tyr Ile Asp Leu Gly Ser Ile Thr Gly
       1580                1585                1590
   Thr Leu Thr Ala Gly Thr Ser Ser Leu Thr Val Leu Asn Asn Arg
       1595                1600                1605
   Val Phe Ala Gly Phe Ala Lys Ser Ser Asn Asp Gly Ile Gly Leu
       1610                1615                1620
   Phe Gly Gly Leu Asn Ala Pro Asp Phe Gly Phe Val Thr Phe Asn
       1625                1630                1635
   Ser Ala Asp Ser Gly Thr Gly Phe Cys Thr Pro Gly Ser Asn Cys
       1640                1645                1650
   Asp Ala Phe Asp Gly Thr Lys Gly Lys Arg Ile Arg Ile Asp Phe
       1655                1660                1665
   Leu Pro Tyr Phe Gly Gly Pro Ser Thr Gly Leu Leu Gly Ile Asn
       1670                1675                1680
   Asn Asn Ala His Pro Asn Trp Ala Tyr Tyr Ile Gly Val Asp Ser
       1685                1690                1695
   Met Phe Val Phe Lys Asn Arg Ile Tyr Ala Ala Asn Gly Gly Leu
       1700                1705                1710
   His Ala Val Gly His Asn Gly Ser Ile Ile Arg Ser Thr Thr Ala
       1715                1720                1725
   Asp Pro Thr Ala Ala Cys Thr Gly Pro Asp Ser Cys Ser Asn Trp
       1730                1735                1740
   Val Glu  Ile Gly Pro Arg Thr  Asn Thr Lys Trp His  Asn Ser Pro
       1745                 1750                 1755
   Thr Asn  Asn Trp Phe Ser Leu  Glu Leu Asn Gln Phe  Tyr Asn Leu
       1760                 1765                 1770
   Ile Pro  Gly Asp Lys Ala Phe  Ala Gln Phe Ala Glu  Phe Asn Asn
       1775                 1780                 1785
   Asn Leu  Tyr Val Thr Arg Thr  Ile Cys Ile Gln Ser  Ser Gln Ala
       1790                 1795                 1800
   Thr Gly  Ile Arg Thr Asn Pro  Gly Thr Val Thr Gly  Cys Thr Asp
       1805                 1810                 1815
   Gly Thr  Thr Thr Asn Arg Arg  Ala Gln Leu Trp Lys  Cys Asp Pro
       1820                 1825                 1830
   Thr Ile  Ser Gly Asn Thr Ser  Glu Cys Asp Ala Ala  Asp Trp Ser
       1835                 1840                 1845
   Val Val  Gly Asp Asp Gly Thr  Gly Ile Thr Asn Met  Gly Asp Ser
       1850                 1855                 1860
   Thr Asn  Arg Thr Ile Thr Met  Val Met Lys Asn Gly  Ser Tyr Leu
       1865                 1870                 1875
   Tyr Ile  Gly Tyr Asp Asn Pro  Asn Gly Ile Arg Ile  Tyr Arg Thr
       1880                 1885                 1890
   Asn Val  Ala Asn Pro Gly Ser  Ser Ser Ala Ser Trp  Ser Gln Ile
       1895                 1900                 1905
   Ala Gly  Asn Gly Leu Thr Asp  Ala Thr Asn Val Gln  Gln Ile Tyr
       1910                 1915                 1920
   Ser Ala  Val Ser Val Pro Ser  Gly Ser Ile Asn Tyr  Ile Tyr Val
       1925                 1930                 1935
   Ser Ala  Gly Lys Ser Asn Val  Ser Val Arg Thr Tyr  Arg Gln Gln
       1940                 1945                 1950
   Asn
<210> 5
   <211> 5658
   <212> DNA
   <213> Leptospira kirschneri
<400> 5
   atgaagagaa cattttgtat ttcgattctt ctttcgatgt tttttcaaag ttgtatgtct 60
   tggccacttt taaccagtct cgcgggttta gcagctggta aaaaaagtaa tgggctgccc 120
   tttttccacc ttctattaag taactctgat ccagttatta caaggatcga gctcagttat 180
   caaaattctt ccatcgcaaa aggtacaagt acaactctcg aagtcaccgc aatctttgat 240
   aacggaacaa atcagaatat tacggattcg acatctatcg tttccgatgc ccaatcaatc 300
   gttgacattc aaggtaacag agtcagagga atcgcttctg gttcttccat tataaaagct 360
   gaatacaacg ggatgtattc tgaacaaaaa attacggtta caccagccac gataaactca 420
   attcaagtta cgagtttaga tgacggtata ttacctaaag gtacaaatcg tcaatttgct 480
   gccatcggta tcttttcgga tggttctcat caagatattt ccaacgatcc attgatcgtt 540
   tggtcttcca gtaatataga tttagttcga gtagatgatt ccggtttggc ctcaggtatc 600
   aatttaggaa cggctcatat tcgtgcatcc tttcaatcaa aacaagcctc cgaagagata 660
   actgttggtg acgctgttct ttcttctatc caagtaactt ccaacagtcc aaatattcct 720
   ctcggaaaaa aacaaaaact cacagctact ggaatttatt cggataactc taacagggat 780
   atttcctctt ctgttatctg gaattcttct aattccacta tcgctaatat tcagaataac 840
   ggaatattag aaacagctga tactggaatt gttactgttt ctgcttctag aggtaatata 900
   aatggttcca taaaactaat cgtcactcct gctgccttag tttctatttc tgtttctcct 960
   acaaattctg cagtagcaaa aggtttacaa gaaaacttta aagctacagg gatctttaca 1020
   gataattcga actcagatat tacagatcaa gttacttggg attcttctaa tccggatatt 1080
   ctttccattt ccaatgcaag tgatagccac gggttagctt ccacactcaa ccaaggaaat 1140
   gttaaggtca ccgcttccat cggtggaata caaggatcca ctgattttaa agttacacaa 1200
   gaggtattaa cttccatcga agtttctcca gttttacctt caattgcaaa aggactaact 1260
   cagaaattta cggcgatcgg gatttttacg gataactcca aaaaagatat tacaaatcaa 1320
   gtcacttgga attcttcttc agcaatcgca agcgtgtcta acttagatga taataaaggt 1380
   ctgggaaaag ctcacgctgt tggagacacg actattaccg ctactttagg aaaagtttca 1440
   ggtaaaactt ggtttactgt agttcctgcg gttctcactt ctattcaaat caatcctgta 1500
   aatccttctc ttgcaaaagg gttaactcaa aaatttacgg ctactgggat ctactctgac 1560
   aactctaaca aggacattac ttcctccgtt acttggttct catccgattc ttcaatcgca 1620
   acaatttcaa acgccaaaaa aaatcaagga aactcttacg gagcagctac aggagcaacg 1680
   gatattaaag ccacattcgg aaaggtaagt agtccagttt ctacgttatc cgttactgct 1740
   gcaaaacttg ttgaaataca aatcacaccg gccgctgctt ccaaagcaaa gggaatttcc 1800
   gaaagattta aagcaaccgg tatttttaca gacaactcta attccgatat tacaaatcag 1860
   gtcacttgga gttcatctaa tacagatatt gctgaaatta caaataccag aggaagcaaa 1920
   ggtattacaa atacactcac tcccggatcg agtgaaatat ccgccgctct cggttcaatc 1980
   aaaagttcta aagtaatatt gaaggtaact ccggcacaat tgatttccat tgcagtaaca 2040
   cctacaaatc catcagttgc aaaaggtcta atacgacaat ttaaagccac cggaacatat 2100
   acggatcatt ccgtacaaga cgtgactgcc ctagctacct ggtcttcttc caatcccaga 2160
   aaagcaatgg ttaacaacgt tacaggttcg gttacaacag tggctaccgg aaatacaaat 2220
   attaaagcaa cgatagactc catatccgga tcttccgttt tgaatgtcac tcctgcactt 2280
   cttacttcta tcgagataac accgacgatt aactctatca ctcacggtct tacaaaacaa 2340
   tttaaagcga ctggtatctt ttcagataaa tctactcaaa atttgactca gcttgtaact 2400
   tggatttctt ccgatccctc caagatcaag atcgaaaata actccggtat agcaacagct 2460
   tctgcattag gaagttcgaa tattacggcc atctacaaat ttgtccaaag ttccccaatt 2520
   ccgatcacag tcactgactt aaaactgaaa agtataacta tcagtccttc ctcaagttca 2580
   atagccaaag gattgaccca acaatttaaa gcgatcggaa cttttataga tggttctgaa 2640
   caagaaatta cgaatcttgt gacctggtat tcctccaaat ccgatattgt tcctatcaat 2700
   aattctgcgg gtaaaaaagg tttagcgacc gcactctcaa taggttcctc caacatctcc 2760
   gcaatttaca attctataag cagtaataaa ataaatttta atgtaagcgc cgccacgtta 2820
   gattccatta aaatcaatcc agtcaacaat aacatcgcca agggacttac ccaacaatat 2880
   actgcgcttg gcgtttattc agactccacc attcaggaca tcagcgattt agttacatgg 2940
   tccagttcca attctgactc gatcagcatc tccaattcga ccggaaccaa gggaaaagcg 3000
   accgctttac agattggaaa gagcaaaatt accgcgactt acaattccat ttcgaaaaac 3060
   ataaatctaa ctgtcagcgc agcaactctc tcttcgattt ttatatctcc taccaataca 3120
   aatataaaca ccaccgtatc aaaacaattc tttgcaatgg gaacgtattc ggacggaacc 3180
   aaaacggatt taacttcttc ggttacatgg tccagttcga atcaagctca agcaaaggtg   3240
   agtaacgcat ctgaaacgaa aggattggtt acagggatta cttctggaaa tcctataatc   3300
   acagcgacct acggctcagt gtcgggaaat acaattctca cagtaaacaa aaccgacacg 3360
   atagctccga cggttcaatc ggtagtttct ttatcaccta ctaccatcca agttgtatat 3420
   tcagaatcca taaacaatca ggaagccctt gatttatcca attacaaaat aattaatagt 3480
   tccaattttt acggacattg ttcggataat acggacttca attccaattc tcaaaccgca 3540
   gatttttctc ttagtagtat caaaggaagt aaaaatactt ttacgattac actttcacat 3600
   tcacaaatct taaacaaatc atacacactt gtagtcaaca aacaaggaat tcacgatctt 3660
   tcttccattc caaattcctt aagttgtcca aataactctg attttatagg aaaagaacaa 3720
   ctcaaactta caagtgcagt ttgtaattcc ttaaaccaag tgatcgtttc tttttccaaa 3780
   cctttatatt ctggaaagga agtaacaaaa tccgtggaat gttcaaatcc gtcccaatgt 3840
   gaatccagat ataaatttgc aggtgtgtct tcattgggaa gtattacgag cgttagaatt 3900
   ttagatggaa aagtatgcgg tggagcaccg gcagactcct cgaaaatatg tttaacacac 3960
   tcccttcttc aatcaggtgg tcaatatacg atcatcgccg caaatgattt gaacggagac 4020
   ggctttgaca acaaatcctg gggagcaatt cgagattcat tcgatcaaga aaacctacaa 4080
   ccttctccga aagatagaat caactttata ggttgtggaa attcccctct caactttatg 4140
   gatggcccga tcgtgtcaga tccttttgga gacggttccg atttcggctc tcttgtagat 4200
   tacaacaatc aaatctatct aggaccgaat gtaaaaggaa accaagcagc tcgattcaat 4260
   tacgacggaa cttttccgga atctattttc ttttctttta cccaagataa aaatgccact 4320
   aaccgtgctt cttcaagaga tggaggaatt ccggttccga attacgttac gatcggtcat 4380
   accggttgta ctctcaatag tgcagacatc actactggat gtggtccaga taacgaagat 4440
   ggacgtgggg tttttgccac cggatcatta gacaaaaaat ctcatatttt tatagcaggt 4500
   tcaaaaccaa ggagattcaa ctatctctat tattcctcag ataccgatac aaaccttaat 4560
   tttaaatata tcagtatggg aaaaattact ggattggcga ctgcaggaac ttcatctatc 4620
   gcagttctag acgatcggat ccatgtaggt tttgcaaaaa aaaatcaaaa tctaaacgca 4680
   cctgatttcg gtaaaatcac ctttaataca tccgagcaca atcgatgtgc aattgtaaac 4740
   aactgtgaag cctctgacgg ataccgcggt aatcgtttta gaatcgatag aatgccttac 4800
   tttggcggcg gctccgtgga tgcagtcaat tataaaactc ataaatctga taattcctcg 4860
   atcaactggg gttattatgt gggaatagat tctctattcg tttttaaaga aaaactttac 4920
   gccgcaaacg gaggatttcc aaattcatta cataatggaa gtataataca ctctaccagt 4980
   gcaaatccta gtccttgtga aggaatcaat cgttgttcca gttggaaaga cacagcacct 5040
   agatccaatc cgaagtggca taactctcct cataccaatt ggttttcact ggagcttaca 5100
   aagtatcgag atttaattcc ggcggataaa gcattctctc aattcgcaga atttaacgga 5160
   agattgtatg taacaagaac gatctgtgta acgaaagaag atcactccgg actcagacaa 5220
   agtttacaaa ctttgaaagg ttgtacagac ggaagttata caaatcgaag acctcaactt 5280
   tggaaatgtg atccgactct aaccggcgat acaacaacct gcgaagcaaa agattggtct 5340
   ttagtaggag ataatggaac cgggtttacg aatttcggag acgattccaa tcacagtatg 5400
   acgatggtag ttgcaagtgg atcttatctc tacgtaggtt ttgacaacga aaacggaatt 5460
   caaatctgga gaacaaatct tgaaaatcct ggaagttcat cacacgactg ggagcctata 5520
   ggaataggcg gattaagaga cgttaccaat cgtcaaattt attcggctat atccggaatg 5580
   aattttggtg taaatttcgt atatataagc gtaggaaata aagatcaacc ggttaaaatt 5640
   tacagacaac agaaccaa 5658
<210> 6
   <211> 1886
   <212> PRT
   <213> Leptospira kirschneri
<400> 6
   Met Lys Arg Thr Phe Cys Ile Ser Ile Leu Leu Ser Met Phe Phe Gln
   1               5                   10                  15
   Ser Cys Met Ser Trp Pro Leu Leu Thr Ser Leu Ala Gly Leu Ala Ala
               20                  25                  30
   Gly Lys Lys Ser Asn Gly Leu Pro Phe Phe His Leu Leu Leu Ser Asn
           35                  40                  45
   Ser Asp Pro Val Ile Thr Arg Ile Glu Leu Ser Tyr Gln Asn Ser Ser
       50                  55                  60
   Ile Ala Lys Gly Thr Ser Thr Thr Leu Glu Val Thr Ala Ile Phe Asp
   65                  70                  75                  80
   Asn Gly Thr Asn Gln Asn Ile Thr Asp Ser Thr Ser Ile Val Ser Asp
                   85                  90                  95
   Ala Gln Ser Ile Val Asp Ile Gln Gly Asn Arg Val Arg Gly Ile Ala
               100                 105                 110
   Ser Gly Ser Ser Ile Ile Lys Ala Glu Tyr Asn Gly Met Tyr Ser Glu
           115                 120                 125
   Gln Lys Ile Thr Val Thr Pro Ala Thr Ile Asn Ser Ile Gln Val Thr
       130                 135                 140
   Ser Leu Asp Asp Gly Ile Leu Pro Lys Gly Thr Asn Arg Gln Phe Ala
   145                 150                 155                 160
   Ala Ile Gly Ile Phe Ser Asp Gly Ser His Gln Asp Ile Ser Asn Asp
                   165                 170                 175
   Pro Leu Ile Val Trp Ser Ser Ser Asn Ile Asp Leu Val Arg Val Asp
               180                 185                 190
   Asp Ser Gly Leu Ala Ser Gly Ile Asn Leu Gly Thr Ala His Ile Arg
           195                 200                 205
   Ala Ser Phe Gln Ser Lys Gln Ala Ser Glu Glu Ile Thr Val Gly Asp
       210                 215                 220
   Ala Val Leu Ser Ser Ile Gln Val Thr Ser Asn Ser Pro Asn Ile Pro
   225                 230                 235                 240
   Leu Gly Lys Lys Gln Lys Leu Thr Ala Thr Gly Ile Tyr Ser Asp Asn
                   245                 250                 255
   Ser Asn Arg Asp Ile Ser Ser Ser Val Ile Trp Asn Ser Ser Asn Ser
   260 265 270
   Thr Ile Ala Asn Ile Gln Asn Asn Gly Ile Leu Glu Thr Ala Asp Thr
   275 280 285
   Gly Ile Val Thr Val Ser Ala Ser Arg Gly Asn Ile Asn Gly Ser Ile
   290 295 300
   Lys Leu Ile Val Thr Pro Ala Ala Leu Val Ser Ile Ser Val Ser Pro
   305 310 315 320
   Thr Asn Ser Ala Val Ala Lys Gly Leu Gln Glu Asn Phe Lys Ala Thr
   325 330 335
   Gly Ile Phe Thr Asp Asn Ser Asn Ser Asp Ile Thr Asp Gln Val Thr
   340 345 350
   Trp Asp Ser Ser Asn Pro Asp Ile Leu Ser Ile Ser Asn Ala Ser Asp
   355 360 365
   Ser His Gly Leu Ala Ser Thr Leu Asn Gln Gly Asn Val Lys Val Thr
   370 375 380
   Ala Ser Ile Gly Gly Ile Gln Gly Ser Thr Asp Phe Lys Val Thr Gln
   385 390 395 400
   Glu Val Leu Thr Ser Ile Glu Val Ser Pro Val Leu Pro Ser Ile Ala
   405 410 415
   Lys Gly Leu Thr Gln Lys Phe Thr Ala Ile Gly Ile Phe Thr Asp Asn
   420 425 430
   Ser Lys Lys Asp Ile Thr Asn Gln Val Thr Trp Asn Ser Ser Ser Ala
   435 440 445
   Ile Ala Ser Val Ser Asn Leu Asp Asp Asn Lys Gly Leu Gly Lys Ala
   450 455 460
   His Ala Val Gly Asp Thr Thr Ile Thr Ala Thr Leu Gly Lys Val Ser
   465 470 475 480
   Gly Lys Thr Trp Phe Thr Val Val Pro Ala Val Leu Thr Ser Ile Gln
   485 490 495
   Ile Asn Pro Val Asn Pro Ser Leu Ala Lys Gly Leu Thr Gln Lys Phe
   500 505 510
   Thr Ala Thr Gly Ile Tyr Ser Asp Asn Ser Asn Lys Asp Ile Thr Ser
   515 520 525
   Ser Val Thr Trp Phe Ser Ser Asp Ser Ser Ile Ala Thr Ile Ser Asn
       530                 535                 540
   Ala Lys Lys Asn Gln Gly Asn Ser Tyr Gly Ala Ala Thr Gly Ala Thr
   545                 550                 555                 560
   Asp Ile Lys Ala Thr Phe Gly Lys Val Ser Ser Pro Val Ser Thr Leu
                   565                 570                 575
   Ser Val Thr Ala Ala Lys Leu Val Glu Ile Gln Ile Thr Pro Ala Ala
               580                 585                 590
   Ala Ser Lys Ala Lys Gly Ile Ser Glu Arg Phe Lys Ala Thr Gly Ile
           595                 600                 605
   Phe Thr Asp Asn Ser Asn Ser Asp Ile Thr Asn Gln Val Thr Trp Ser
       610                 615                 620
   Ser Ser Asn Thr Asp Ile Ala Glu Ile Thr Asn Thr Arg Gly Ser Lys
   625                 630                 635                 640
   Gly Ile Thr Asn Thr Leu Thr Pro Gly Ser Ser Glu Ile Ser Ala Ala
                   645                 650                 655
   Leu Gly Ser Ile Lys Ser Ser Lys Val Ile Leu Lys Val Thr Pro Ala
               660                 665                 670
   Gln Leu Ile Ser Ile Ala Val Thr Pro Thr Asn Pro Ser Val Ala Lys
           675                 680                 685
   Gly Leu Ile Arg Gln Phe Lys Ala Thr Gly Thr Tyr Thr Asp His Ser
       690                 695                 700
   Val Gln Asp Val Thr Ala Leu Ala Thr Trp Ser Ser Ser Asn Pro Arg
   705                 710                 715                 720
   Lys Ala Met Val Asn Asn Val Thr Gly Ser Val Thr Thr Val Ala Thr
                   725                 730                 735
   Gly Asn Thr Asn Ile Lys Ala Thr Ile Asp Ser Ile Ser Gly Ser Ser
               740                 745                 750
   Val Leu Asn Val Thr Pro Ala Leu Leu Thr Ser Ile Glu Ile Thr Pro
           755                 760                 765
   Thr Ile Asn Ser Ile Thr His Gly Leu Thr Lys Gln Phe Lys Ala Thr
       770                 775                 780
   Gly Ile Phe Ser Asp Lys Ser Thr Gln Asn Leu Thr Gln Leu Val Thr
   785 790 795 800
   Trp Ile Ser Ser Asp Pro Ser Lys Ile Lys Ile Glu Asn Asn Ser Gly
   805 810 815
   Ile Ala Thr Ala Ser Ala Leu Gly Ser Ser Asn Ile Thr Ala Ile Tyr
   820 825 830
   Lys Phe Val Gln Ser Ser Pro Ile Pro Ile Thr Val Thr Asp Leu Lys
   835 840 845
   Leu Lys Ser Ile Thr Ile Ser Pro Ser Ser Ser Ser Ile Ala Lys Gly
   850 855 860
   Leu Thr Gln Gln Phe Lys Ala Ile Gly Thr Phe Ile Asp Gly Ser Glu
   865 870 875 880
   Gln Glu Ile Thr Asn Leu Val Thr Trp Tyr Ser Ser Lys Ser Asp Ile
   885 890 895
   Val Pro Ile Asn Asn Ser Ala Gly Lys Lys Gly Leu Ala Thr Ala Leu
   900 905 910
   Ser Ile Gly Ser Ser Asn Ile Ser Ala Ile Tyr Asn Ser Ile Ser Ser
   915 920 925
   Asn Lys Ile Asn Phe Asn Val Ser Ala Ala Thr Leu Asp Ser Ile Lys
   930 935 940
   Ile Asn Pro Val Asn Asn Asn Ile Ala Lys Gly Leu Thr Gln Gln Tyr
   945 950 955 960
   Thr Ala Leu Gly Val Tyr Ser Asp Ser Thr Ile Gln Asp Ile Ser Asp
   965 970 975
   Leu Val Thr Trp Ser Ser Ser Asn Ser Asp Ser Ile Ser Ile Ser Asn
   980 985 990
   Ser Thr Gly Thr Lys Gly Lys Ala Thr Ala Leu Gln Ile Gly Lys Ser
   995 1000 1005
   Lys Ile Thr Ala Thr Tyr Asn Ser Ile Ser Lys Asn Ile Asn Leu
   1010 1015 1020
   Thr Val Ser Ala Ala Thr Leu Ser Ser Ile Phe Ile Ser Pro Thr
   1025 1030 1035
   Asn Thr Asn Ile Asn Thr Thr Val Ser Lys Gln Phe Phe Ala Met
   1040 1045 1050
   Gly Thr Tyr Ser Asp Gly Thr Lys Thr Asp Leu Thr Ser Ser Val
   1055 1060 1065
   Thr Trp Ser Ser Ser Asn Gln Ala Gln Ala Lys Val Ser Asn Ala
   1070 1075 1080
   Ser Glu Thr Lys Gly Leu Val Thr Gly Ile Thr Ser Gly Asn Pro
   1085 1090 1095
   Ile Ile Thr Ala Thr Tyr Gly Ser Val Ser Gly Asn Thr Ile Leu
   1100 1105 1110
   Thr Val Asn Lys Thr Asp Thr Ile Ala Pro Thr Val Gln Ser Val
   1115 1120 1125
   Val Ser Leu Ser Pro Thr Thr Ile Gln Val Val Tyr Ser Glu Ser
   1130 1135 1140
   Ile Asn Asn Gln Glu Ala Leu Asp Leu Ser Asn Tyr Lys Ile Ile
   1145 1150 1155
   Asn Ser Ser Asn Phe Tyr Gly His Cys Ser Asp Asn Thr Asp Phe
   1160 1165 1170
   Asn Ser Asn Ser Gln Thr Ala Asp Phe Ser Leu Ser Ser Ile Lys
   1175 1180 1185
   Gly Ser Lys Asn Thr Phe Thr Ile Thr Leu Ser His Ser Gln Ile
   1190 1195 1200
   Leu Asn Lys Ser Tyr Thr Leu Val Val Asn Lys Gln Gly Ile His
   1205 1210 1215
   Asp Leu Ser Ser Ile Pro Asn Ser Leu Ser Cys Pro Asn Asn Ser
   1220 1225 1230
   Asp Phe Ile Gly Lys Glu Gln Leu Lys Leu Thr Ser Ala Val Cys
   1235 1240 1245
   Asn Ser Leu Asn Gln Val Ile Val Ser Phe Ser Lys Pro Leu Tyr
   1250 1255 1260
   Ser Gly Lys Glu Val Thr Lys Ser Val Glu Cys Ser Asn Pro Ser
   1265 1270 1275
   Gln Cys Glu Ser Arg Tyr Lys Phe Ala Gly Val Ser Ser Leu Gly
   1280 1285 1290
   Ser Ile Thr Ser Val Arg Ile Leu Asp Gly Lys Val Cys Gly Gly
   1295 1300 1305
   Ala Pro Ala Asp Ser Ser Lys Ile Cys Leu Thr His Ser Leu Leu
   1310 1315 1320
   Gln Ser Gly Gly Gln Tyr Thr Ile Ile Ala Ala Asn Asp Leu Asn
   1325 1330 1335
   Gly Asp Gly Phe Asp Asn Lys Ser Trp Gly Ala Ile Arg Asp Ser
   1340 1345 1350
   Phe Asp Gln Glu Asn Leu Gln Pro Ser Pro Lys Asp Arg Ile Asn
   1355 1360 1365
   Phe Ile Gly Cys Gly Asn Ser Pro Leu Asn Phe Met Asp Gly Pro
   1370 1375 1380
   Ile Val Ser Asp Pro Phe Gly Asp Gly Ser Asp Phe Gly Ser Leu
   1385 1390 1395
   Val Asp Tyr Asn Asn Gln Ile Tyr Leu Gly Pro Asn Val Lys Gly
   1400 1405 1410
   Asn Gln Ala Ala Arg Phe Asn Tyr Asp Gly Thr Phe Pro Glu Ser
   1415 1420 1425
   Ile Phe Phe Ser Phe Thr Gln Asp Lys Asn Ala Thr Asn Arg Ala
   1430 1435 1440
   Ser Ser Arg Asp Gly Gly Ile Pro Val Pro Asn Tyr Val Thr Ile
   1445 1450 1455
   Gly His Thr Gly Cys Thr Leu Asn Ser Ala Asp Ile Thr Thr Gly
   1460 1465 1470
   Cys Gly Pro Asp Asn Glu Asp Gly Arg Gly Val Phe Ala Thr Gly
   1475 1480 1485
   Ser Leu Asp Lys Lys Ser His Ile Phe Ile Ala Gly Ser Lys Pro
   1490 1495 1500
   Arg Arg Phe Asn Tyr Leu Tyr Tyr Ser Ser Asp Thr Asp Thr Asn
   1505 1510 1515
   Leu Asn Phe Lys Tyr Ile Ser Met Gly Lys Ile Thr Gly Leu Ala
   1520 1525 1530
   Thr Ala Gly Thr Ser Ser Ile Ala Val Leu Asp Asp Arg Ile His
   1535 1540 1545
   Val Gly Phe Ala Lys Lys Asn Gln Asn Leu Asn Ala Pro Asp Phe
   1550 1555 1560
   Gly Lys Ile Thr Phe Asn Thr Ser Glu His Asn Arg Cys Ala Ile
   1565 1570 1575
   Val Asn Asn Cys Glu Ala Ser Asp Gly Tyr Arg Gly Asn Arg Phe
   1580 1585 1590
   Arg Ile Asp Arg Met Pro Tyr Phe Gly Gly Gly Ser Val Asp Ala
   1595 1600 1605
   Val Asn Tyr Lys Thr His Lys Ser Asp Asn Ser Ser Ile Asn Trp
   1610 1615 1620
   Gly Tyr Tyr Val Gly Ile Asp Ser Leu Phe Val Phe Lys Glu Lys
   1625 1630 1635
   Leu Tyr Ala Ala Asn Gly Gly Phe Pro Asn Ser Leu His Asn Gly
   1640 1645 1650
   Ser Ile Ile His Ser Thr Ser Ala Asn Pro Ser Pro Cys Glu Gly
   1655 1660 1665
   Ile Asn Arg Cys Ser Ser Trp Lys Asp Thr Ala Pro Arg Ser Asn
   1670 1675 1680
   Pro Lys Trp His Asn Ser Pro His Thr Asn Trp Phe Ser Leu Glu
   1685 1690 1695
   Leu Thr Lys Tyr Arg Asp Leu Ile Pro Ala Asp Lys Ala Phe Ser
   1700 1705 1710
   Gln Phe Ala Glu Phe Asn Gly Arg Leu Tyr Val Thr Arg Thr Ile
   1715 1720 1725
   Cys Val Thr Lys Glu Asp His Ser Gly Leu Arg Gln Ser Leu Gln
   1730 1735 1740
   Thr Leu Lys Gly Cys Thr Asp Gly Ser Tyr Thr Asn Arg Arg Pro
   1745 1750 1755
   Gln Leu Trp Lys Cys Asp Pro Thr Leu Thr Gly Asp Thr Thr Thr
   1760 1765 1770
   Cys Glu Ala Lys Asp Trp Ser Leu Val Gly Asp Asn Gly Thr Gly
   1775 1780 1785
   Phe Thr  Asn Phe Gly Asp Asp  Ser Asn His Ser Met  Thr Met Val
       1790                 1795                 1800
   Val Ala  Ser Gly Ser Tyr Leu  Tyr Val Gly Phe Asp  Asn Glu Asn
       1805                 1810                 1815
   Gly Ile  Gln Ile Trp Arg Thr  Asn Leu Glu Asn Pro  Gly Ser Ser
       1820                 1825                 1830
   Ser His  Asp Trp Glu Pro Ile  Gly Ile Gly Gly Leu  Arg Asp Val
       1835                 1840                 1845
   Thr Asn  Arg Gln Ile Tyr Ser  Ala Ile Ser Gly Met  Asn Phe Gly
       1850                 1855                 1860
   Val Asn  Phe Val Tyr Ile Ser  Val Gly Asn Lys Asp  Gln Pro Val
       1865                 1870                 1875
   Lys Ile  Tyr Arg Gln Gln Asn  Gln
       1880                 1885
<210> 7
   <211> 1557
   <212> DNA
   <213> Leptospira kirschneri
<400> 7
   attaccgtta caccagccat tcttaactca attcaagtta cgagtttaga gtcaggtata      60
   ctacctaaag gtactaatcg tcaattctca gccatcggta tcttttcgga tggttctcat     120
   caggatattt ccaacgaacc actgatcgtt tggtcttcca gtaatcctga tttggttcga     180
   gtagatgatt cagggttggc atcagggatc aatttaggaa cagctcatat tcgtgcatcc     240
   tttcaatcaa aacaaggggc tgaagaaatg accgttggag atgctgttct ctctcaaatc     300
   caagtaactt caaacgatct gaatattcct ctcggaaaaa aacaaaaact aacagctacg     360
   ggaatctatt cggataactc taacagggat atttcctctt ctgttatttg gaattcttct     420
   aattccacta tcgctaatat tcaaaacaac ggaatattag aaacagctga tactggtatt     480
   gtcactgttt ctgcttctag cgagaatata atcggatccg taaaactaat cgttactcca     540
   gcagccttag tttctatttc tgtttctccg acaaattcta cagttgcaaa aggtttacaa     600
   gaaaacttta aagctacagg gatctttaca gataattcaa actcggatat taccgaccaa     660
   gttacttggg attcttctaa taccgatatt ctctcaattt ccaatgcaag tgatagccac     720
   ggattagctt ccacactcaa ccaagggaat gttaaagtca ctgcttccat cggtggaata     780
   caaggatcca ctgattttaa agttacacaa gctgcattga cttccatcga agtctctcca     840
   actcgcactt ccattgcaaa aggactaact caaaagttta ctgcgatcgg gatttttacg     900
   gataactcta agaaggatat tacggatcaa gtcacttgga attcttcttc agcaatcgta     960
   agcgtgtcta acttagacaa caataaaggt ctgggaaaaa ccaactcagt tggaaacacg    1020
   actattaccg caaccttagg aaaagtttca ggtaacactt ggtttactgt agttcctgcg    1080
   gttctcactt ctattcaaat caatcctgta aatccttctc ttgcaaaagg gttaactcaa    1140
   aaatttacgg ctactgggat ctactctgac aactctaaca aggacattac ttccgctgtt    1200
   acgtggttct catccgattc ttcaatcgcg acgatttcaa acgcccaaaa aaatcaagga    1260
   aacgcttacg gagcagctac aggagcaacg gatattaaag ccacattcgg aaaggtaagt    1320
   agtccggttt ctacgttatc tgttacagct gcaaagcttg ttgaaatcca aatcacaccg    1380
   gctgctgctt ccaaagcaaa gggactcaca gaaagattca aggctactgg tatctttacg    1440
   gataactcaa attccgatat tacaaatcaa gttacctgga attcctctaa tacggatatt    1500
   gctgaaatta aaaataccag tggaagtaaa ggtattacaa atacactcac tccagga       1557
<210> 8
   <211> 519
   <212> PRT
   <213> Leptospira kirschneri
<400> 8
   Ile Thr Val Thr Pro Ala Ile Leu Asn Ser Ile Gln Val Thr Ser Leu
   1               5                   10                  15
   Glu Ser Gly Ile Leu Pro Lys Gly Thr Asn Arg Gln Phe Ser Ala Ile
               20                  25                  30
   Gly Ile Phe Ser Asp Gly Ser His Gln Asp Ile Ser Asn Glu Pro Leu
           35                  40                  45
   Ile Val Trp Ser Ser Ser Asn Pro Asp Leu Val Arg Val Asp Asp Ser
       50                  55                  60
   Gly Leu Ala Ser Gly Ile Asn Leu Gly Thr Ala His Ile Arg Ala Ser
   65                  70                  75                  80
   Phe Gln Ser Lys Gln Gly Ala Glu Glu Met Thr Val Gly Asp Ala Val
                   85                  90                  95
   Leu Ser Gln Ile Gln Val Thr Ser Asn Asp Leu Asn Ile Pro Leu Gly
               100                 105                 110
   Lys Lys Gln Lys Leu Thr Ala Thr Gly Ile Tyr Ser Asp Asn Ser Asn
           115                 120                 125
   Arg Asp Ile Ser Ser Ser Val Ile Trp Asn Ser Ser Asn Ser Thr Ile
       130                 135                 140
   Ala Asn Ile Gln Asn Asn Gly Ile Leu Glu Thr Ala Asp Thr Gly Ile
   145                 150                 155                 160
   Val Thr Val Ser Ala Ser Ser Glu Asn Ile Ile Gly Ser Val Lys Leu
   165 170 175
   Ile Val Thr Pro Ala Ala Leu Val Ser Ile Ser Val Ser Pro Thr Asn
   180 185 190
   Ser Thr Val Ala Lys Gly Leu Gln Glu Asn Phe Lys Ala Thr Gly Ile
   195 200 205
   Phe Thr Asp Asn Ser Asn Ser Asp Ile Thr Asp Gln Val Thr Trp Asp
   210 215 220
   Ser Ser Asn Thr Asp Ile Leu Ser Ile Ser Asn Ala Ser Asp Ser His
   225 230 235 240
   Gly Leu Ala Ser Thr Leu Asn Gln Gly Asn Val Lys Val Thr Ala Ser
   245 250 255
   Ile Gly Gly Ile Gln Gly Ser Thr Asp Phe Lys Val Thr Gln Ala Ala
   260 265 270
   Leu Thr Ser Ile Glu Val Ser Pro Thr Arg Thr Ser Ile Ala Lys Gly
   275 280 285
   Leu Thr Gln Lys Phe Thr Ala Ile Gly Ile Phe Thr Asp Asn Ser Lys
   290 295 300
   Lys Asp Ile Thr Asp Gln Val Thr Trp Asn Ser Ser Ser Ala Ile Val
   305 310 315 320
   Ser Val Ser Asn Leu Asp Asn Asn Lys Gly Leu Gly Lys Thr Asn Ser
   325 330 335
   Val Gly Asn Thr Thr Ile Thr Ala Thr Leu Gly Lys Val Ser Gly Asn
   340 345 350
   Thr Trp Phe Thr Val Val Pro Ala Val Leu Thr Ser Ile Gln Ile Asn
   355 360 365
   Pro Val Asn Pro Ser Leu Ala Lys Gly Leu Thr Gln Lys Phe Thr Ala
   370 375 380
   Thr Gly Ile Tyr Ser Asp Asn Ser Asn Lys Asp Ile Thr Ser Ala Val
   385 390 395 400
   Thr Trp Phe Ser Ser Asp Ser Ser Ile Ala Thr Ile Ser Asn Ala Gln
   405 410 415
   Lys Asn Gln Gly Asn Ala Tyr Gly Ala Ala Thr Gly Ala Thr Asp Ile
               420                 425                 430
   Lys Ala Thr Phe Gly Lys Val Ser Ser Pro Val Ser Thr Leu Ser Val
           435                 440                 445
   Thr Ala Ala Lys Leu Val Glu Ile Gln Ile Thr Pro Ala Ala Ala Ser
       450                 455                 460
   Lys Ala Lys Gly Leu Thr Glu Arg Phe Lys Ala Thr Gly Ile Phe Thr
   465                 470                 475                 480
   Asp Asn Ser Asn Ser Asp Ile Thr Asn Gln Val Thr Trp Asn Ser Ser
                   485                 490                 495
   Asn Thr Asp Ile Ala Glu Ile Lys Asn Thr Ser Gly Ser Lys Gly Ile
               500                 505                 510
   Thr Asn Thr Leu Thr Pro Gly
           515
<210> 9
   <211> 600
   <212> DNA
   <213> Leptospira kirschneri
<400> 9
   cataactctc ctcataacaa ttggttttca ctggagctta caaagtatcg gaatttaatt      60
   ccggcggata aagcattctc tcaattcgca gaatttaacg gaagattgta tgtaacaaga     120
   acgatctgcg taacgaaaga agatcactcc ggactcagac aaagtttaca aactgtggaa     180
   ggttgtacgg acggaagtta tacaaatcga agaccccaac tttggaaatg tgatccgact     240
   ctaaccggcg atacaacaac ctgcgaagca gaagattggt ctttagtagg agataacgga     300
   accggattta caaactttgg agacaattcc aatcacagta tgacgatgat ggttgcaagt     360
   ggatcttatc tctacatagg ttttgataac gaaaacggaa ttcaaatctg gagaacaaat     420
   cttgaaaatc ctggaagttc atcacacaac tgggaaccta taggaatagg cggattaaga     480
   gacgttacca atcgtcaaat ttattcggct atatccggaa tgaattttgg tgtaaatttc     540
   gtatatataa gcgtaggaaa caaaaataaa ccggtcaaaa tttacagaca acagaatcaa     600
<210> 10
   <211> 200
   <212> PRT
   <213> Leptospira kirschneri
<400> 10
   His Asn Ser Pro His Asn Asn Trp Phe Ser Leu Glu Leu Thr Lys Tyr
   1 5 10 15
   Arg Asn Leu Ile Pro Ala Asp Lys Ala Phe Ser Gln Phe Ala Glu Phe
   20 25 30
   Asn Gly Arg Leu Tyr Val Thr Arg Thr Ile Cys Val Thr Lys Glu Asp
   35 40 45
   His Ser Gly Leu Arg Gln Ser Leu Gln Thr Val Glu Gly Cys Thr Asp
   50 55 60
   Gly Ser Tyr Thr Asn Arg Arg Pro Gln Leu Trp Lys Cys Asp Pro Thr
   65 70 75 80
   Leu Thr Gly Asp Thr Thr Thr Cys Glu Ala Glu Asp Trp Ser Leu Val
   85 90 95
   Gly Asp Asn Gly Thr Gly Phe Thr Asn Phe Gly Asp Asn Ser Asn His
   100 105 110
   Ser Met Thr Met Met Val Ala Ser Gly Ser Tyr Leu Tyr Ile Gly Phe
   115 120 125
   Asp Asn Glu Asn Gly Ile Gln Ile Trp Arg Thr Asn Leu Glu Asn Pro
   130 135 140
   Gly Ser Ser Ser His Asn Trp Glu Pro Ile Gly Ile Gly Gly Leu Arg
   145 150 155 160
   Asp Val Thr Asn Arg Gln Ile Tyr Ser Ala Ile Ser Gly Met Asn Phe
   165 170 175
   Gly Val Asn Phe Val Tyr Ile Ser Val Gly Asn Lys Asn Lys Pro Val
   180 185 190
   Lys Ile Tyr Arg Gln Gln Asn Gln
   195 200

## Description

### FIELD OF THE INVENTION

The invention relates to three isolated DNA molecules that encode for proteins, BigL1, BigL2 and BigL3, in the *Leptospira sp* bacterium which have repetitive Bacterial-Ig-like (Big) domains and their use in diagnostic, therapeutic and vaccine applications. According to the present invention, the isolated molecules encoding for BigL1, BigL2 and BigL3 proteins are used for the diagnosis and prevention of infection with *Leptospira* species that are capable of producing disease in humans and other mammals, including those of veterinary importance.

### BACKGROUND OF THE INVENTION

Spirochetes are motile, helically shaped bacteria and include three genuses, *Leptospira, Borrelia* and *Treponema,* which are pathogens of humans and other animals. *Borrelia* and *Treponema* are the causative agents of diseases that include Lyme disease, relapsing fever, syphilis and yaws. *Leptospira* consists of a genetically diverse group of eight pathogenic and four non-pathogenic, saprophytic species (1, 2). Leptospires are also classified according to serovar status - more than 200 pathogenic serovars have been identified. Structural heterogeneity in lipopolysaccharide moieties appears to be the basis for the large degree of antigenic variation observed among serovars (1, 2).

Leptospirosis is a, zoonotic disease: transmission to humans occurs through contact with domestic or wild animal reservoirs or an environment contaminated by their urine. Infection produces a wide spectrum of clinical manifestations. The early-phase of illness is characterized by fever, chills, headache and severe myalgias. Disease progresses in 5 to 15% of the clinical infections to produce severe multisystem complications such as jaundice, renal insufficiency and hemorrhagic manifestations (1-4). Severe leptospirosis is associated with mortality rates of 5-40%.

Leptospirosis has a world-wide distribution. Because of the large spectrum of animal species that serve as reservoirs, it is considered to be the most widespread zoonotic disease (1). Leptospirosis is traditionally an important occupational disease among risk groups such as military personnel, farmers, miners, sewage and refuse removal workers, veterinarians and abattoir workers (1-3). However, new patterns of disease transmission have emerged recently that emphasize the growing importance of leptospirosis as a public health problem. In developed countries, leptospirosis has become the cause of outbreaks associated with recreational activities (1) and sporting events (1, 4, 5). In Brazil and other developing countries, underlying conditions of poverty have produced large urban epidemics of leptospirosis associated with high mortality (4, 5).

In addition to its public health impact, leptospirosis is a major economic burden as the cause of disease in livestock and domestic animals (2). Leptospirosis produces abortions, stillbirths, infertility, failure to thrive, reduced milk production and death in animals such as cows, pigs, sheep, goats, horses and dogs and induces chronic infection and shedding of pathogenic leptospires in livestock (2) and therefore represents an additional source of economic loss for the animal husbandry industry because of current international and national quarantine regulations.

The control of human and animal leptospirosis is hindered by the current lack of adequate diagnostic tools. The standard serologic test, the microscopic agglutination test (MAT), is inadequate for rapid case identification since it can only be performed in few reference laboratories and requires analyses of paired sera to achieve sufficient sensitivity (1, 2). Dependence upon the MAT results in delays in establishing the cause of outbreaks as seen in several investigations (1, 2). Enzyme-linked immunosorbent assays (ELISA), and other rapid serologic tests based on whole-cell leptospiral antigen preparations have been developed for use as an alternative method to screen for leptospiral infection, although the MAT is still required for case confirmation (1, 2). Recombinant antigen-based serologic tests are widely used in screening for spirochetal infections such as Lyme disease and syphilis, but the use of recombinant proteins for serodiagnosis of leptospirosis has not been widely investigated. Recently, a recombinant flagellar-antigen immuno-capture assay was described for serodiagnosis of bovine leptospirosis (6). A recombinant heat shock protein, Hsp58, showed a high degree of ELISA reactivity with serum samples from a small number of human cases (7). However, the utility of recombinant antigens for the serodiagnosis of leptospirosis has not been investigated in large validation studies.

Furthermore, there are no effective interventions presently available, which control or prevent leptospirosis. Environmental control measures are difficult to implement because of the long-term survival of pathogenic leptospires in soil and water and the abundance of wild and domestic animal reservoirs (1, 3). Efforts have focused on developing protective immunization as an intervention against leptospirosis. Currently-available vaccines are based on inactivated whole cell or membrane preparations of pathogenic leptospires and appear to induce protective responses through induction of antibodies against leptospiral lipopolysaccharide (1, 3). However, these vaccines do not induce long-term protection against infection. Furthermore, they do not provide cross-protective immunity against leptospiral serovars that are not included in the vaccine preparation. The large number of pathogenic serovars (>200) and the cost of producing a multi-serovar vaccine have been major limitations in developing efficacious vaccines through strategies based on whole cell or membrane preparations.

The mechanism of pathogenesis in leptospirosis, as in spirochetal disease such as Lyme disease and syphilis, relies on the pathogen's ability to widely disseminate within the host during the early stage of infection (2). Membrane-associated leptospiral proteins are presumed to mediate interactions that enable entry and dissemination through host tissues. Putative surface-associated virulence factors serve as candidates for vaccine strategies that induce responses to these factors which block dissemination in the host. Furthermore, membrane-associated proteins would be accessible to the immune response during host infection and therefore, constitute targets for immune protection through mechanisms such antibody-dependent phagocytosis and complement-mediated killing. Production of these antigen targets as recombinant proteins offers a cost-effective approach for protective immunization for leptospirosis as a sub-unit based vaccine. In addition, selection of surface-associated targets that are conserved among pathogenic leptospires can avoid the limitations encountered with currently available whole-cell vaccine preparations.

A major limitation in the field of leptospirosis has been identifying surface-associated and host-expressed proteins with conventional biochemical and molecular methods. From the genome sequence of the spirochete, *Borrelia burgdorferi,* more than 100 surface associated lipoproteins were identified. Based on genome size and the biology of its lifecycle, *Leptospira* are expected to have a significantly greater number of surface-associated targets. At present, less than 10 surface-associated proteins have been characterized though isolation of membrane extracts, purification and characterization of proteins in these extracts and molecular cloning of these protein targets (8-14) (12). Immunization with recombinant proteins for several identified targets, LipL32, OmpL1 and LipL41, induce partial, but not complete, protective responses (11, 12).

To develop a more comprehensive understanding of leptospiral protein expression we have used the humoral immune response during human leptospirosis as a reporter of protein antigens expressed during infection. The identification of leptospiral antigens expressed during infection has potentially important implications for the development of new serodiagnostic and immunoprotective strategies. Sera from patients with leptospirosis was used to identify clones from a genomic *Leptospira* DNA phage library which express immunoreactive polypeptides. A proportion of these clones were found to encode a novel family of membrane-associated *Leptospira* proteins. The identification of these polynucleotides and polypeptides and their application for diagnosis of leptospirosis and inducing an immune response to pathogenic spirochetes is the basis for this invention.

### SUMMARY OF THE INVENTION

The invention relates to DNA molecules in *Leptospira* and the polypeptides they encode which have repetitive bacterial Ig-like domains. The invention describes the isolation of three DNA molecules, originally derived from *L*. *kirschneri* and *L. interrogans,* which encode proteins, herein designated "BigL1" "BigL2" and "BigL3" , that have molecular masses of approximately 110, 205 and 205 kDa, respectively, based on the predicted amino acid sequence of the polypeptides. The three proteins have 12-13 tandem repeat sequences of approximately 90 amino acids. Repeats sequence from. BigL1, BigL2 and BigL3 are highly related (>90% amino acid sequence identify) to each other and belong to the family of bacteria Ig-like (Big) domains, moieties which are found in virulence factors of bacterial pathogens.

The DNA molecules that encode for *Leptospira* proteins with Big domains, herein called "*bigL1*", "*bigL2*" and "*bigL3*", can be inserted as heterologous DNA into an expression vector for producing peptides and polypeptides. Recombinant polypeptides can be purified from surrogate hosts transformed with such expression vectors. BigL1, BigL2 and BigL3-derived polypeptides are serological markers for active and past infection since sera from leptospirosis patients and animals infected or immunized with pathogenic *Leptospira* recognize isolated polypeptides.

Furthermore, BigL1, BigL2 and BigL3 polypeptides from recombinant or native antigen preparations are immunogenic. Antibodies obtained from experimental animals immunized with purified recombinant BigL1, BigL2 and BigL3 polypeptides recognize native antigen from *Leptospira,* and are useful for detecting pathogenic spirochetes in samples from subjects with a suspected infection.

In addition, BigL1, BigL2 and BigL3 polypeptides induce an immune response against pathogenic spirochetes. BigL1, BigL2 and BigL3-derived polypeptides; antibodies to these polypeptides; and polynucleotides that encode for BigL1, BigL2 and BigL3 may be used alone or combined with pharmaceutically acceptable carrier to treat or prevent infection with *Leptospira*. Since Big domains are present in proteins associated with virulence in other bacterial pathogens, these moieties may be used to treat or prevent infections unrelated to those caused by *Leptospira.*

In a first embodiment, the disclosure describes isolated DNA molecules for *bigL1, bigL2* and *bigL3* and the polypeptides that are encoded by these DNA molecules. In addition, a method is provided for producing an expression vector containing *bigL1, bigL2* and *bigL3* polynucleotides and obtaining substantially purified polypeptides derived from these sequences.

A second embodiment of the present disclosure is to describe pharmaceutical composition for inducing immune responses in subjects to pathogenic spirochetes, comprising of an immunogenically effective amount of one or more selected antigens among the group consisting of BigL1, BigL2, BigL3 and polypeptides with functionally equivalent sequences in a pharmaceutically acceptable vehicle.

In a third embodiment, the disclosure describes a method for identifying a compound which binds specifically to BigL1, BigL2, BigL3 polypeptides or polypeptides that includes incubating components comprising of the compound and BigL1, BigL2 or BigL3 polypeptide or polypeptides under conditions sufficient to allow the components to interact and measuring the binding of the compound to the BigL1, BigL2 or BigL3 polypeptide or polypeptides. Preferably, the inventive method is a serodiagnostic method utilizing sera from a subject with a suspected active or past infection with *Leptospira* or other related bacterial pathogens.

In a fourth embodiment, the disclosure describes a method for detecting pathogens in a sample which includes contacting a sample suspected of containing a pathogenic spirochete with a reagent that binds specifically to the pathogen-specific cell component and detecting binding of the reagent to the component. In another aspect, the reagent that specifically binds to the pathogen-specific cell component is an antibody against the BigL1, BigL2 or BigL3 polypeptide or polypeptides.

A fifth embodiment, the disclosure describes a kit useful for the detection of BigL1, BigL2, and BigL3 polypeptides or polypeptides or antibodies that bind specifically to BigL1, BigL2, BigL3, polypeptides or polypeptides.

Other objects, features and advantages of the present disclosure will become apparent from the following detailed description.

### BRIEF DESCRIPTION OF THE DRAWINGS

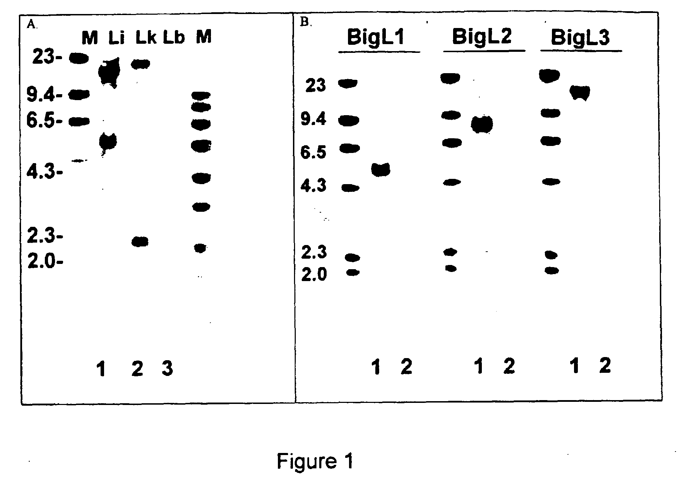

FIGS. 1A and B show a Southern blot analysis of *bigL* gene sequences in *Leptospira.* Genomic DNA (3mcg/lane) from *L. interrogans* strain Fiocruz L1-130 (lanes 1), *L. kirschneri* strain Rm52 (lanes 2) and *L. biflexi* strain Patoc I (lanes 3) digested with NsiI and subject to agarose gel electrophoresis. After transfer to nitrocellulose membranes, blots were probed with DNA fragments that encode for BigL repetitive domains (4^{th}-6^{th} repetitive domain of BigL3, FIG. 1A) and C-terminal regions of *bigL1, bigL2* and *bigL3,* which are unique to each of these genes, respectively (FIG.1B).

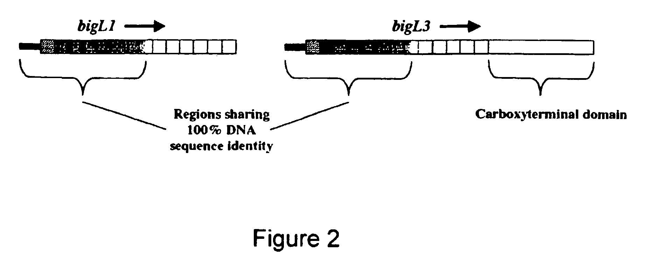

FIG. 2 shows a schematic diagram of the genomic organization of the region encoding the BigL1 and BigL3 proteins in *L. kirschneri*. The BigL1 protein would contain a signal peptide (hatched box) and thirteen 90-amino-acid bacterial immunoglobulin-like domains (solid boxes). The BigL3 protein would contain a signal peptide, twelve 90-amino-acid bacterial immunoglobulin-like domains, and a 793 amino acidcarboxyterminal (C-terminal) domain. The locations of the 2156 bp region of 100% DNA sequence identity are shown. The organization of the region depicted was conserved in *L. interrogans* and *L. kirschneri.*

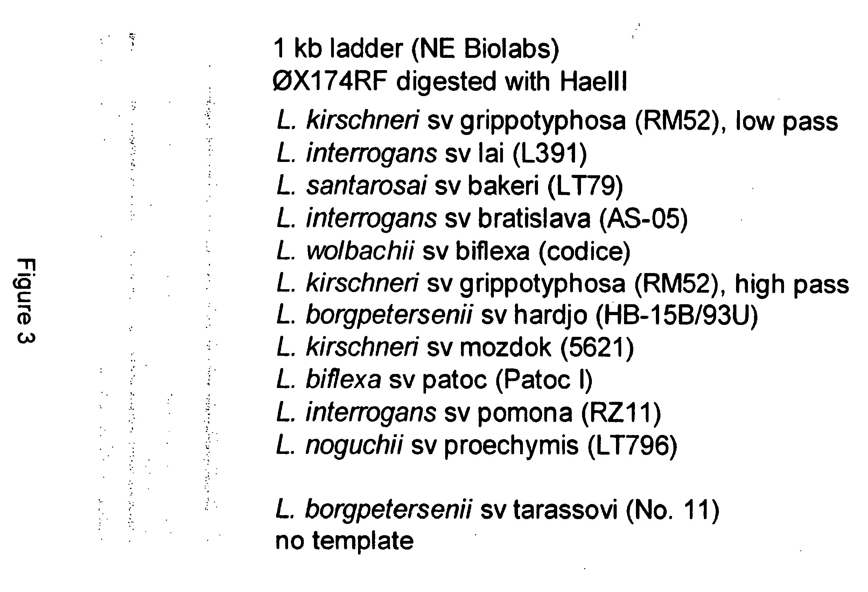

FIG. 3 shows the polymerase chain reaction (PCR) amplification of DNA fragments from strains of five pathogenic species of *Leptospira.* Degenerate primers were designed based on the *L. kirschneri* and *L. interrogans* sequence encoding for the BigL3 region corresponding to positions 46-65 aa. PCR reactions were performed from purified DNA from five pathogenic (*L. kirschneri, borgpetersenii, interrogans, santarosai,* and *noguchi*) and two non-pathogenic species (*L. biflexi* and *wolbachii*).

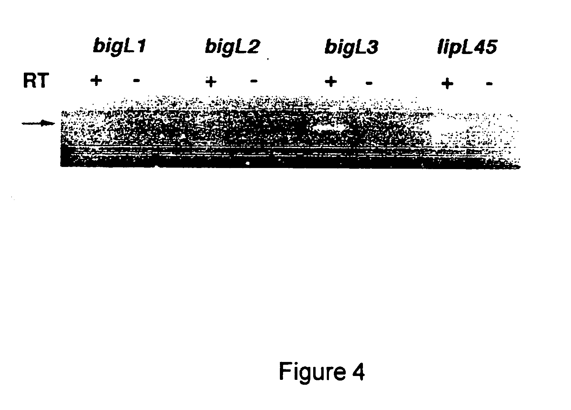

FIG. 4 shows amplified products from RT-PCR of RNA extracts of *L. kirschneri* with *bigL1, bigL2* and *bigL3* specific primers. Reverse transcription reactions (lanes "+") were performed on RNA extracts of cultured leptospires and then subject to a polymerase chain reaction (PCR) amplification step with primers that bind to unique sequences within *bigL1, bigL2* and *bigL3.* Amplification with primers based on sequences within *lipL45* was performed as a control reaction as was PCR reactions for which samples were not subjected to the reverse transcription step.

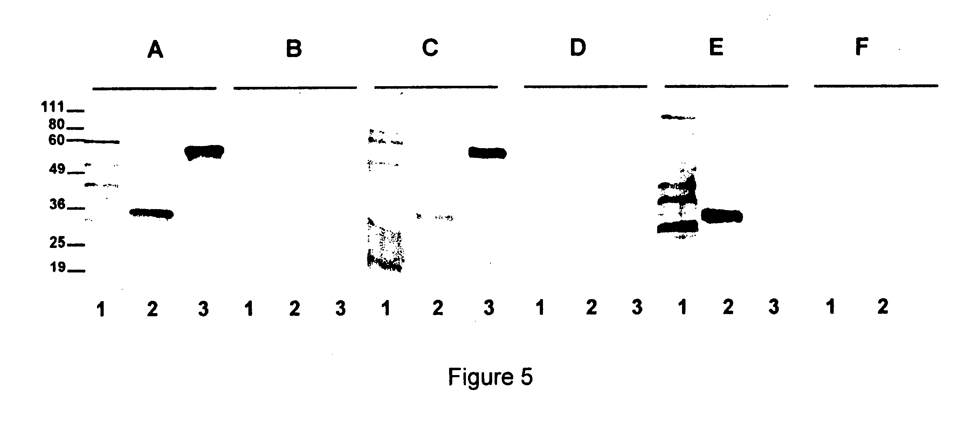

FIG. 5 shows the immunoblot reactivity of pooled sera from patients and animal reservoirs infected with pathogenic *Leptospira* and laboratory animals immunized with whole *L. interrogans* antigen preparation to recombinant BigL3 protein (rBigL3). Western blot analysis was performed with purified rBigL3 (1mcg per lane, lanes 3). Membranes were probed with sera from patients with leptospirosis (lane A), healthy individuals (lane B), captured rats that are colonized with *L. interrogans* (lane C), captured rats that are not colonized with *L. interrogans* (lane D), laboratory rats immunized with whole antigen preparations of in vitro cultured *L. interrogans* (lane E) and pre-immune sera from the laboratory rats collected prior to immunization (lane F). Reactivity to whole *L. interrogans* antigen preparation (lanes 1) and recombinant LipL32 protein (rLipL32, lanes 2) is shown for comparison. The numbers on the left indicate the positions and relative mobilities (kDa) for molecular mass standards (Invitrogen).

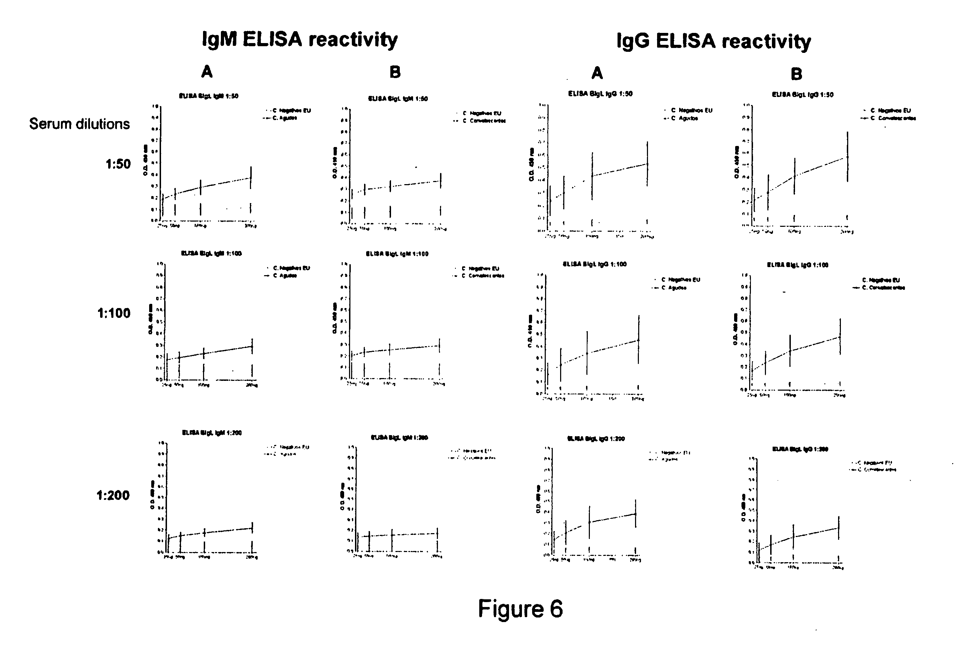

FIG. 6 shows an ELISA evaluation of individual patient seroreactivity to rBigL3 during the acute (lanes A) and convalescent (lanes B) phase of illness with leptospirosis. Sera from 4 leptospirosis patients (unbroken lines) and 4 health individuals (broken lines), at dilutions of 1:50, 1:100 and 1:200, were incubated with RBigL3 (25-200ng/well). Mu and gamma chain specific antibodies conjugated to horse radish peroxidase was used to determine IgM and IgG seroreactivity, respectively. Mean absorbance values (OD 450nm) and standard deviations are represented in the graphs.

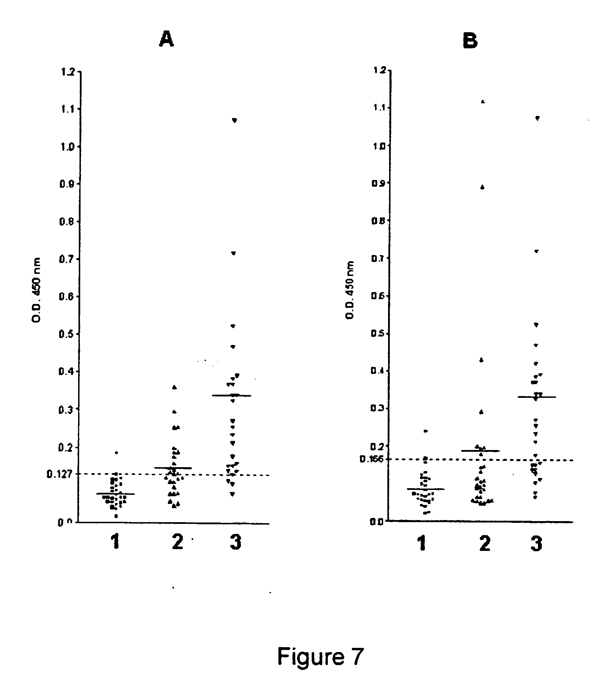

FIG. 7 shows the rBigL3 IgM (Column A) and IgG (Column B) reactivity of sera from 29 individual patients with leptospirosis during the acute (lanes 2) and convalescent (lanes 3) phase of illness and 28 health individuals (lanes 1). Sera (1:50 dilutions) and Mu and gamma chain specific antibodies conjugated to horse radish peroxidase were used to determine reactivity. Solid bars represent mean absorbance (OD 450nm) values.

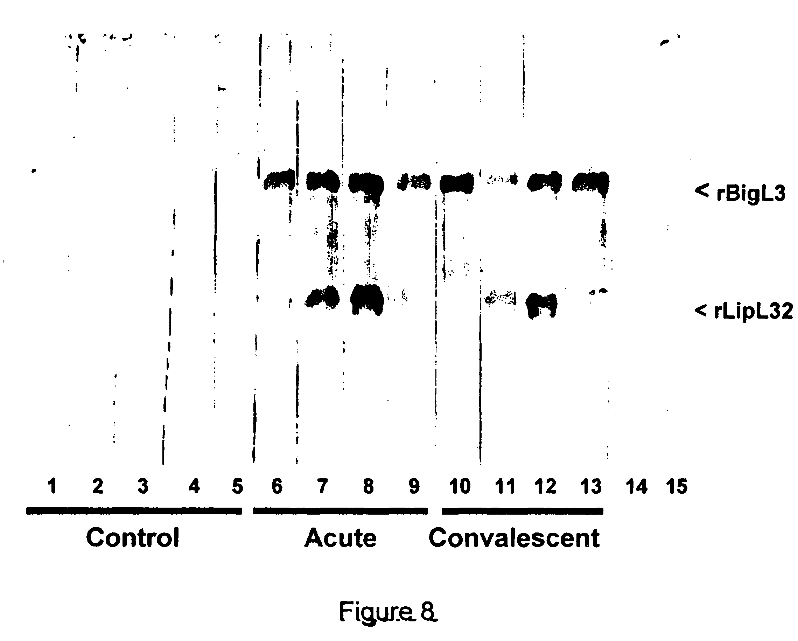

FIG. 8 shows the immunoblot reactivity of individual patients with leptospirosis to rBigL3 during the acute (lanes 6-9) and convalescent (lanes 10-13) phase of illness. Western blot analysis was performed with purified rBigL3 (1mcg per lane, lanes 3). Membranes were probed with sera diluted 1:100. A gamma chain-specific antibodies conjugated to alkaline phosphatase were used to determine reactivity to the recombinant 58 kD protein of region 1 of BigL3 (2^{nd} to 6^{th} Big repeat domains). Reactivity to rLipL32 (1 mcg per lane) was performed as a comparison. The mobility of purified rBigL32 and rLipL32 (lane 14) and molecular mass standards (lane 15) are shown after staining with Ponceau-S and coomassie blue, respectively.

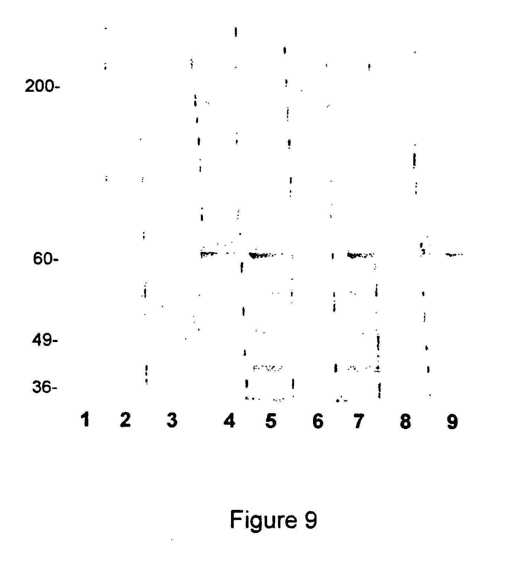

FIG. 9 shows the immunoblot reactivity of rat anti-rBigL3 antisera to rBigL3 and native angigen from *L. interrogans* lysates. Immunoblots were prepared with purified rBigL3 (1mg/lane; lanes 3, 5, 7, 9) and whole antigen preparations (10⁸ leptospira per lane; lanes 2, 4, 6 and 8) from cultured leptospires. Membranes were probed with pooled sera (dilutions 1:500 [lanes 4 and 5], 1:100 [lanes 6 and 7] and 1:2500 [lanes [8 and 9]] from rats immunized with rBigL3 from E. coli ewpressing a cloned DNA fragment of *bigL3* from *L. interrogans.* Pre-immune sera was obtained prior to the first immunization and used in the immunoblot analysis as a control (lanes 2 and 3). The mobility (kDa) of molecular mass standards are shown on the left side of the figure

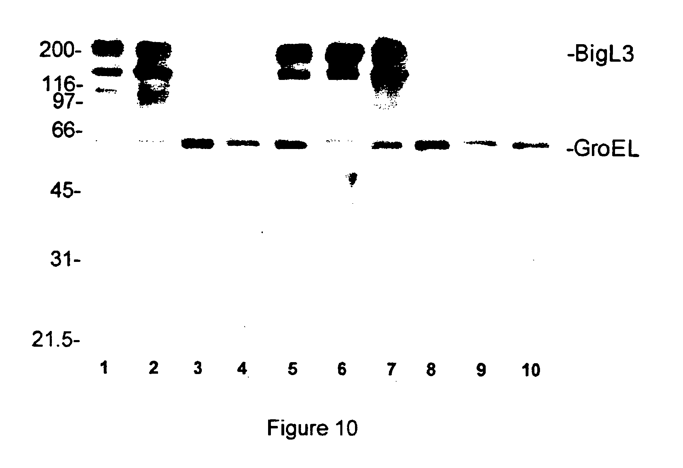

FIG. 10 shows the immunoblot reactivity of rabbit anti-rBigL3 antisera to native antigen from *Leptospira* strain lysates. Immunoblots were prepared with whole antigen preparations (10⁸ leptospira per lane) of the following cultured strains: lane 1, *L interrogans* sv pomona (type kennewicki) strain RM211, low-passage; lane 2, *L*. *interrogans* sv canicola strain CDC Nic 1808, low passage; lane 3, *L. interrogans* sv pomona strain PO-01, high passage; lane 4, *L. interrogans* sv bratislava strain AS-05, high passage; lane 5, *L. kirschneri* sv grippotyphosa strain RM52, low passage; lane 6, *L. kirschneri* sv grippotyphosa strain P8827-2, low passage; lane 7, *L*. *kirschneri* sv grippotyphosa strain 86-89, low passage; lane 8, *L. kirschneri* sv grippotyphosa strain Moskva V, high passage; lane 9, *L*. *kirschneri* sv mozdok strain 5621, high passage; lane 10, *L. kirschneri* sv grippotyphosa strain RM52, high passage. Membranes were probed with sera from rabbits immunized with rBigL3 from *E*. *coli* expressing a cloned DNA fragment of *bigL3* from *L. kircshneri* and, as a control measure, sera from rabbits immunized with recombinant L. kirschneri GroEL protein. The positions of native antigens corresponding to BigL3 and GroEL and the mobility (kDa) of molecular mass standards are shown on the left and right sides, respectively, of the figure.

### DETAILED DESCRIPTION OF THE INVENTION

For convenience, the meaning of certain terms and phrases employed in the specification, examples, and appended claims are provided below. Unless defined otherwise, all technical and scientific terms used herein have the same meaning as commonly understood by one of ordinary skill in the art to which this invention belongs.

BigL - are polypeptides of *Leptospira sp.* having tandem repeat sequences each of which are similar, according to their sequence homology, to bacterial immunoglobulin-like (Big) domains. Big domains are present in bacterial proteins, expressed in bacterial pathogens such as *E*. *coli, Yersinia* and *Bordetella,* which have virulence functions such as host cell adhesion.

Reference sequence - is a new sequence obtained by isolation from a natural organism or through genetic engineering and presents an accurate biological function, which is characteristic of the present invention.

Functionally equivalent sequences - are the sequences, related to a reference sequence, that are the result of variability, i.e. all modification, spontaneous or induced, in a sequence, being substitution and/or deletion and/or insertion of nucleotides or amino acids, and/or extension and/or shortening of the sequence in one of their ends, without resulting in modification of the characteristic function of the reference sequence. Functionally equivalent sequences emcompass fragment and analog therof. In other words, sequences functionally equivalent are sequences that are "substantially the same" or "substantially identical" to the reference sequence, such as polypeptides or nucleic acids that have at least 80% homology in relation to the sequence of amino acids or reference nucleic acids. The homology usually is measured by a software system that performs sequence analyses (Sequence Analysis Software Package of the Genetics Computer Group, University of Wisconsin Biotechnology Center, 1710, University Avenue, Madison, Wis, 53705).

As we mentioned before, *Leptospira* antigens expressed during the host infection are important in the identification of targets for diagnosis tests and vaccines. The LipL32 protein is one of these targets and was identified as immunodominant antigen by the immune humoral response during the natural infection. However the sensitivity of serologic tests based upon detection of antibodies against LipL32 in patient sera during acute-phase illness with leptospirosis detection is limited (see Flannery, B: "Evaluation of recombinant Leptospira antigen-based Enzyme-linked Immunosorbent Assays for the serodiagnosis of Leptospirosis" J.Clin. Microbiology 2001;39(9): 3303-3310; WO9942478).

The present invention is based on the identification of the family of proteins BigL associated with species of spirochetal bacteria, including those belonging to *Leptospira.*

According to the present invention, the BigL protein family was identified as targets of the host humoral immune response, generated during infection with pathogenic *Leptospira* or immunization with pathogenic Leptospira or recombinant BigL polypeptides. BigL polypeptides and polynucleotides that encode these polypeptides are useful as in diagnostic tests to identify naturally occurring infection in different species including humans and animal reservoirs. The diagnostic test based on those proteins presents improved sensitivity and specificity in relation to standard diagnostic tests or those that are have been used in the published literature. the identification of leptospirosis in the initial phase. In addition BigL polypeptides can induce immune responses when used in a pharmaceutical composition for immunization.

In the present invention, the three BigL polypeptides are characterized with molecular weights 128.4 kD, 201.3 kD and 200.4kD, based on the deduced amino acid sequence of the isolated polynucleotides, *bigL1*, *bigL2* and *bigL3,* which encode for these polypeptides. The amino acid sequence of the BigL polypeptides has a signal sequence and a putative signal peptidase cleavage site largely conforming to the spirochetal lipobox; therefore BigL polypeptides are membrane-associated lipoproteins. The polypeptides of 128.4 kD, 201.3 kD and 200.4 kD are designated "BigL1" "BigL2" and "BigL3", respectively..

Although the BigL polypeptides of the present invention have been isolated originally of *Leptospira sp,* they are useful not just for induction of the immune response against the pathogenic organisms *Leptospira sp.,* but also against other spirochetes bacteria and pathogens that have factors with Big domains. Additionally, BigL polypeptides can be used for the diagnosis of infections due to *Leptospira sp.,* other pathogenic spirochetes and bacterial pathogens.

Several processes that incorporate state-of-the-art methodologies can be used to obtain polynucleotide sequences that encode for BigL polypeptides. These processes include, but they are not limited to, the isolation of DNA using hybridization of genomic libraries with probes to detect homologous sequences of nucleotides; screening of antibodies of expression libraries to detect fragments of cloned DNA with shared structural aspects; polymerase chain reaction (PCR) in genomic DNA using initiators able to recombine sequence of DNA of interest; and computer-based searches of sequence databases for similar sequences to that of the *bigL* polynucleotides.

In the present invention the identification of the antigens was based on knowledge that there is differential expression of *Leptospira* antigens during culture (*in vitro*) and during host infection (*in vivo*). Differential expression of *Leptospira* antigens is presumed to be important in host adaptation during infection. We used a strategy to identify immunoreactive antigens and therefore antigens expressed during host infection. Sera from patients infected with pathogenic *Leptospira* were used to select polynucleotide sequences from genomic *Leptospira* DNA library in lambda phage that encode for immunoreactive polypeptides.

The present invention identified and isolated three polynucleotides with nucleotide sequences corresponding to SEQ ID No:1, SEQ ID No:3 and SEQ ID No:5, as well as the amino acid sequences of the respective polypeptides, BigL1, BigL2 and BigL3, encoded by such nucleotides.
**Step 1** - The screening the positive clones consisted basically of the following steps:
   (a) The DNA of a pathogenic *Leptospira* was cut with an appropriate enzyme and ligated into a specific site in the lambda phage genome. Host bacteria were infected with phage and the resulting clones, expressing recombinant polypeptides after induction with IPTG, were submitted to immunoblot protocol where a membrane of colony lysates was incubated with sera from patients with laboratory confirmed leptospirosis and then with a secondary antibody conjugated to horseradish peroxidase, which recognized human Ig. Positive clones were detected through an indicator reaction, for antigen-antibody complexes based on the production of color.
   (b) The sequence of cloned and isolated polynucleotides was determined using phage vector-specific sequences as initiators of the sequencing reaction. Analysis of the clone sequences and the use of a primer walking strategy identified the complete nucleotide sequence for the genes that encode for BigL1, BigL2, and BigL3.
   (c) Most of the obtained positive clones contains genes encoding proteins of thermal shock *Hsp58* and *DnaK* and the protein of outer membrane LipL41. However, it was found clones containing genes encoding repetitions in tandem of 90 amino acids compared by Database of proteins family (Pfam) as proteins of bacterium, type immunoglobulin (Big). With the analysis of the clone sequences, were identified 3 genes containing 12 tandem repeats for bigL1 and 13 tandem repeats in bigL2 and bigL3.
**Step 2** - Subcloning expression and purification of the protein
   - Drawing of two oligonucleotides with base in sequences of two proteins BigL
   - Amplification by PCR of the initial BigL portion encoding for part of the repetitive region, from those oligonucleotides
   - Sequencing of the product of the amplification
   - Subcloning of the region-encoding by the product sequenced
   - Expression of the recombinant protein.
   - Purification of the recombinant protein.

Immunoblot analyses demonstrate that sera from leptospirosis patient and rodent reservoirs infected with pathogenic *Leptospira* produce antibodies primarily to the BigL domain repeats of the BigL polypeptides, indicating that they are the main antigenic regions recognized during infection.

In relation to the polypeptides of the present invention they consist of sequences of DNA, cDNA or RNA (and sequences of nucleic acids which are complementary), as well as their functionally equivalent sequence, i.e., those sequences that encode the whole or a part, of the polypeptides designated as BigL1, BigL2 and BigL3, but are non-identical due to variability.

The polypeptides and polynucleotides in the present invention consist of BigL1, BigL2 and BigL3 and the polynucleotides that encode these polypeptides; however they include, in addition, polypeptides and polynucleotides that have functionally equivalent sequence.

In the present invention, both polynucleotides and polypeptides may be of natural, synthetic or recombinant origin, having the necessary purity degree to grant to their biological activities.

The present invention also refers to the polynucleotides encoding for BigL1, BigL2 and BigL3 which are used in PCR reactions to obtain either complete or partial amplified DNA fragments of the bigL polynucleotides, for the purpose of detection of Leptospira in samples or expression of recombinant BigL polypeptides. In the case of initiators used for the polynucleotide amplification in the present invention, they are oligonucleotides made of two or more deoxyribonucleotides or ribonucleotides, natural or synthetic.

Each initiator is preferably constructed in order to be substantially similar to a flanking region of the sequence strand that is the target for amplification. In this sense, an initiator can be designated functionally equivalent if corresponding polymers can produce the same process, without being identical, facing the utilization or application considered.

Polynucleotide sequences of this invention can also be inserted in an expression vector, such as a plasmid, virus or other vehicle used for recombinant cloning, which is used by inserting or incorporating whole or partial nucleotide sequences that encode for BigL1, BigL2 and BigL3 or their functionally equivalent sequences. Such expression vectors contain a promoter sequence that facilitates the efficient transcription from genetic sequence in the host in which the vector is inserted. Such hosts can include prokaryotes or eukaryotes, including microorganisms such as yeast or insects and mammals. Such processes for the use of expression vectors construction and for the expression of recombinant sequences, properly so-called, are well known by experts in technique.

The present invention provides for a method to produce antibodies that bind to complete or partial polypeptides of BigL1, BigL2 and BigL3 or their functionally equivalent sequences. Such antibodies are useful as research and diagnostic tools in the study and diagnosis of spirochete infections in general, and more specifically in the development of diagnostics and therapeutics whether treatment or prevention, for leptospirosis. Such antibodies can be administered alone or as part of a pharmaceutical composition that use these antibodies and a pharmaceutically acceptable carrier as part of anti-spirochetal therapeutic.

The invention is relates to the use of pharmaceutical compositions of BigL polypeptides or the polynucleotides that encode for these polypeptides as vaccines, either as a vaccine for prevention of disease which induces an immunoprotective, response to infection or colonization with pathogenic spirochetes or as therapeutic vaccine that provides a beneficial impact in reducing the duration or severity of the clinical course of illness in an subject due infected with a pathogenic spirochete or in reducing the reservoir state of a carrier of pathogenic spirochete such as in pigs, cows, rats or dogs that harbor and excrete pathogenic spirochetes for prolonged periods of time. Such compositions may be prepared with an immunogenically effective quantity of an antibody against BigL1, BigL2 and BigL3 respectively, or with one or more of BigL1, BigL2 and BigL3 isolated from the leptospiral pathogen or recombinant BigL polypeptides, or its functionally equivalent sequences, in excipients and additives or auxiliaries.

Another embodiment of present invention relates to the pharmaceutical composition used to induce an immune response to a *pathogenic* spirochete in an individual, particularly *Leptospira sp.,* including a immunologically effective quantity of BigL1, BigL2 and BigL3 or of their functionally equivalent sequence in a pharmaceutically acceptable vehicle. As "individual" we refer to any mammal, including humans, rodents, domesticated and laboratory animals and livestock. As "quantity immunologically effective" we refer to quantity of BigL polypeptide antigen necessary to induce, in an individual, an immunological response against *Leptospira* or any other pathogenic spirochete or bacterial pathogen. The invention further provides a kit for:
1 - detecting one of polypeptides, BigL1, BigL2 and BigL3, or their functionally equivalent sequences;
2 -detecting nucleic acid encoding for BigL1, BigL2 and BigL3 or their functionally equivalent sequences;
3 - detecting antibodies for such polypeptides, BigL1, BigL2 and BigL3, or their functionally equivalent sequences.

The kit used for detection of BigL polypeptides includes those that use a vehicle containing one or more receptacles with a first receptacle containing a linking reagent to BigL1, BigL2 and BigL3 or to their functionally equivalent sequences.

The kit used for detection of polynucleotides that encode BigL polypeptides includes those that use a vehicle containing one or more receptacles with a first receptacle containing a polynucleotide that hybridizes to the nucleic acid sequence that encodes BigL1, BigL2 and BigL3 or to their functionally equivalent sequences.

The kit useful for detecting antibodies against BigL polypeptides includes those that use a vehicle containing one or more receptacles with a first receptacle containing a polypeptide of BigL1, BigL2 and BigL3 or of their functionally equivalent sequences.

The present invention will be now described with reference to the Examples, which are should not be considered as limitative of the present invention.

### EXAMPLE 1:

### Example 1A: Bacterial strains, plasmids and media

*Leptospira kirschneri* serovar grippotyphosa, strain RM52, was isolated during an outbreak of porcine abortion in 1983). *L. interrogans* serovar copenhageni, strain Fiocruz (L1-130), was isolated from the bloodstream of a human leptospirosis patient. *L. kirschneri* serovar grippotyphosa strain RM52 and other leptospiral strains were obtained from the National Leptospirosis Reference Center (National Animal Disease Center, Agricultural Research Service, U.S. Department of Agriculture, Ames, Iowa). Leptospiral strains were cultivated at 30°C in Johnson-Harris bovine serum albumin-Tween80 medium (Bovuminar PLM-5 Microbiological Media, Intergen (2). Low-passage samples of the RM52 isolate were either stored in liquid nitrogen or passaged in liquid medium at least 200 times to generate a high-passage form. The high-passage strain was unable to produce a lethal infection in hamsters at any dose and was only able to infect hamsters at a dose of 10⁷ by intraperitoneal inoculation.

*Escherichia coli* XL1-Blue MRF'□mcrA)183□mcrCB-hsdSMR-mrr)173 endA1 supE44 thi-1 recA1 gyrA96 relA1 lac [F'proAB lacI^{q}Z□M15 Tn10 (Tetr)] (Stratagene) and E. *coli* PLK-F' (*endA1 gyrA96 hsdR17 lac⁻ recA1 relA1 supE44 thi-1* [F' *lacI^{q}Z□M15*]*)* were used as the host strains for infection with the λZap II (Stratagene) and λTriplEx (Clontech) vectors, respectively. *E. coli* SOLR (e14⁻ [*mcrA*], □[*mcrCB-hsdSMR-mrr*]*171 sbcC recB recJ umuC*::Tn*5*[Kan^{r}] *uvrC lac gyrA96 relA1 thi-1 endA1* λ^{r}, [F' *proAB lacI*^{q}*Z*□M15], Su⁻ [non-suppressing]] and *E. coli* BM25.8 (*supE44 thi* □*lac-proAB* [F' *traD36 proAB*⁺ *lacI^{q}Z*□*M15*] λ*imm434(kan^{r})* P1(*cam^{r}*) *hsdR*(*r*^{K12}*⁻m*^{K12})) were used for in vivo excision of the pBluescript and pTriplEx phagemids, respectively. BLR(DE3)pLysS [F⁻ *ompT hsdS*_{B} (r_{B}-m_{B}-) *gal dcm _(srl-recA) 306::*Tn*10*(TcR) (DE3) pLysS(CmR)] (Novagen) was used as the host strain for the pRSET expression vector (Invitrogen). *E. coli* strains were grown in LB supplemented with 100 µg/ml ampicillin, 100 µg/ml carbenicillin, or 25 µg/ml chloramphenicol where appropriate. Antibiotics were purchased from Sigma.

### Example 1B: Isolation and Characterization of bigL Genes:

This example illustrates the identification and isolation of the *bigL* genes. Genomic DNA was prepared from virulent, low-passage *L. kirschneri,* serovar grippotyphosa, strain RM52 by the method of Yelton and Charon (15). Genomic DNA was prepared from a clinical isolate of *L. interrogans,* serovar copenhageni, strain Fiocruz L1-130, using a kit to genomic DNA (Qiagen). The QIAquick PCR Purification Kit (Qiagen) was used to obtain purified DNA. The genomic DNA was partially digested with Tsp509I and ligated to λTriplEx arms following the instructions provided (Clontech). The Gigapack III Gold Packaging Extract (Stratagene) was used to packaged ligated, digested genomic DNA into lambda heads. The phage titer of the library was determined by infection *E*. *coli* XL1-Blue.

For screening of genomic library, approximately 10³ pfu approximately were plated on a lawn of E. coli XL1-Blue, transferred to nitrocellulose membrane (Schleicher & Schuell), sensibilized with IPTG and processed as recommended (Schleicher & Schuell). The nitrocellulose filter was blocked with 5% skimmed milk in Tris-buffered saline (pH7.8) with 0.05% Tween 20 (TBST) or phosphate-buffered saline (pH 7.4) with 0.05% Tween 20 (PBST), and incubated for 1 hour with pooled sera, diluted 1:50, from patients with laboratory-confirmed leptospirosis. Sera were collected from patients, identified in urban epidemics in Brazil between 1996 and 1999, during the convalescent-phase of illness, and were pre-absorbed with *E*. *coli* lysates prior to use to remove antibodies to *E*. *coli.* Membranes were washed three times with TBST or PBST, and incubated for more than 1 hour with rabbit or goat anti-human immunoglobin antibody conjugated with alkaline phosphatase (Sigma) in the dilution of 1:1000. Detection with NBT (0,3 mg/ml) and BCIP (0,15 mg/ml) or development with the ECL Western Blot Detection Reagents (Amersham) followed by exposure to Hyperfilm (Amersham) was used to identify plaques with antigen-antibody complexes.

Each positive plaque was stored at 4°C in 1 ml SM (0,1 1 M NaCl, 8 mM MgSO₄, 50 mM Tris-HCl pH 7,5; 0,01% in gelatin, with 1-2 drops of chloroform. The lambda plaque clones that reacted with pooled sera were subjected to two additional stages of purification. The genomic DNA fragments inserted into lambda bacteriophage were excised by infecting *E. coli* SOLR or BM25.8 strains with the lambda clones as described by the supplier (Stratagene and Clontech, respectively).

The sequence of the first 500-700 nucleotides of the insert was obtained using a vector-specific primer that links adjacent to the insert. Nucleotide sequence analysis of 131 clones identified 13 that had DNA fragment inserts, found to encode tandem repeats approximately 90 amino acids in length. Each of the repeat sequences were subsequently identified in Pfam 6.6 (http://pfam.wustl.edu/) to belong to the Big2 family Big2 family of bacterial immunoglobulin-like (Big) domains.

To identify sequences that encode full-length proteins according to the predicted amino acid sequence, the nucleotide sequences of the clones were assembled from individual sequences obtained by a combination of primer walking and sequencing of nested deletions. The deletions were generated from the plasmid clones by removal of restriction fragments extending from inside the insert into the multicloning sites flanking the insert. Oligonucleotides were synthesized and obtained from GIBCO BRL or Operon. Inverse PCR (iPCR) was performed to obtain sequences containing the remainder of the genes and flanking DNA. The UCLA Core Sequencing Facility, the Yale/Keck Core DNA Sequencing Facility and the University of California at Berkeley Sequencing Facility performed the sequencing reactions.

Two *L. kirschneri* clones and four *L. interrogans* clones were found to encode a gene which we designate bacterial immunoglobulin-like Leptospiral protein one, *bigL1.* The complete nucleotide sequence of *L. kirschneri bigL1* and the predicted amino acid sequence of the gene product is shown in SEQ ID NO: 1 and SEQ ID NO: 2. Six *L. kirschneri* clones were found to encode a second gene which we designated *bigL2.* The complete nucleotide sequence of *L. kirschneri bigL2* is shown in SEQ ID NO: 3. *L. kirschneri bigL2* appears to be a pseudogene, an extra adenine residue occurs at nucleotide 1011 resulting in a frameshift mutation and downstream TAG stop codon. However, the antibody screening with pooled patient sera was able to identify lamda clones with DNA fragments encoding bigL2 gene products, presumably since the cloned fragments did not have the frameshift mutation and were inserted in an orientation that allowed expression of a product that was recognized by patient sera. The predicted amino acid sequence of the *L. kirschneri bigL2* gene product, without the frameshift mutation, is shown in SEQ ID NO: 4. A fifth *L. interrogans* clone was found to encode several Big repeats initially thought to belong to BigL1. However the upstream DNA encoded by this fifth *L. interrogans* clone was found to differ from the sequence upstream of bigL1. Sequencing the regions flanking the bigL1 gene revealed that the fifth *L. interrogans* clone corresponded to a third gene, designated *bigL3,* downstream of *bigL1* (FIG. 2). The complete nucleotide sequence for *bigL3* was obtained from *L. kirschneri* DNA and is shown in SED ID NO: 5. The predicted amino acid sequence of the *L. kirschneri bigL3* gene product is shown in SEQ ID NO: 6.

All three bigL genes encode a signal peptide and putative signal peptidase cleavage site largely conforming to the spirochetal lipobox, as previously defined (Haake, D. A. 2000. Spirochetal lipoproteins and pathogenesis. Microbiology. 146:1491-1504). Comparison of the sequences of known spirochetal lipoproteins indicates that the spirochetal lipobox is much more loosely defined than the E. coli lipobox. For example, while most E. coli lipoproteins have Leu in the -3 position relative to Cys, spirochetal lipoproteins may also have a number of other hydrophobic amino acids in this position, including Val, Phe, and Ile. *E. coli* experiments involving site-specific mutagenesis of amino acids following cysteine indicates that acidic residues cause sorting of lipoproteins to the cytoplasmic membrane. Sequence analysis of leptospiral lipoproteins indicates that a similar sorting signal is present in these bacteria. For example, LipL31 is the only lipoprotein having an unopposed negative charge in the first two amino acids following cysteine, and is also the only lipoprotein sorted exclusively to the cytoplasmic membrane. Like the outer membrane lipoproteins LipL32 and LipL41, the BigL proteins have uncharged amino acids in the +2 and +3 positions, indicating that they would be sorted to the outer membrane.

Following their signal peptides, all three proteins would contain a series of tandem repeats, approximately 90-amino-acids in length. The mature BigL1 protein would consist almost entirely of thirteen repeats, while in contrast BigL2 and BigL3 contain twelve repeats followed large carboxy-terminal domains. Though there is a high degree of sequence variation among the 31 unique repeats found in the three proteins, all of the repeats were identified by the Pfam database as bacterial immunoglobulin-like Big protein family with E-values as low as 4 x e⁻³⁰.

The *L. interrogans* and *L. kirschneri* versions of bigL1, bigL2, and bigL3 were highly related, with >90% dna and amino acid sequence identity. In both species there is a region of DNA sequence identity involving the 5' ends of *bigL1* and big*L3* (FIG 2). The region of sequence identity begins extends from the initial ATG start codon to position 1890 bp in both genes. The large region of DNA sequence identity between bigL1 and bigL3 results in an identical amino acid sequence for the first 630 amino acids (positions 1-630) of BigL1 (SEQ ID NO: 2) and BigL3 (SEQ ID NO: 6). This region of identity corresponds to the first six BigL domain repeats.

### EXAMPLE 2

### Example 2A: Characterization of the bigL genes and Detection of bigL DNA and RNA

This example illustrates the distribution of multiple copies of *bigL* genes among *Leptospira* species and methods to detect *bigL* DNA and RNA in samples.

### Southern Blot Analysis

Southern blot analysis was performed to identify multiple copies of bigL genes in genomic DNA from *L*. *interrogans* strain Fiocruz L1-130, *L. kirschneri* strain RM52, and *L. biflexi* strain Patoc I. DNA restriction and modifying enzymes were purchased from New England Biolabs. Genomic DNA was extracted from from 500ml of 7-day cultures of *Leptospira* cells with the Blood and Cell Culture kit (Qiagen, Valencia, Calif.). Approximately 3mcg of DNA was digested with 5-20 units of NsiI overnight in a final volume of 50mcl. DNA was then purified with phenol:chloroform:isoamyl and precipitated with 100% cold ethanol and 3M sodium acetate pH and washed with 70% ethanol. Purified DNA was then re-digested with 5-20 units *Pac*I overnight in a final volume of 25 mcl. The double digested DNA was separated in a 0.8% agarose gel at 20V overnight. The gel was then incubated twice for 30 minutes in denaturing buffer (1.5 M NaCl, 0.5 N NaOH), and twice for 30 minutes in neutralization buffer (1M Tris (pH7.4) 1.5 M NaCl). Genomic DNA was transfered onto a positively charged nylon membrane (Roche Molecular Biochemicals, Indianapolis, Ind.) according to the method described by Southern.

Probes were synthesized with the PCR Dig Probe Synthesis kit (Roche, Manheim, Germany). Reactions were assembled according to the manufacturer in a final volume of 50mcl. Temperature cycles for the amplification were 94°C for 5 min, 94 °C for 30 sec, 57 °C for 30 s min, and 72 °C for 1 min, with a final extension time of 7 min after a total of 35 cycles. Probe sequences were as follows: to amplify the *bigL* DNA fragments that encodes for BigL repetitive domains, a *bigL3* DNA sequence was selected that correspond to the region that encodes for BigL3 repetitive domains 4-6, BigL3_395 gat-ttt-aaa-gtt-aca-caa-gc and BigL3_573 aaa-ccg-gac-tac-tta-cct-ttc-c; and to amplify *bigL* DNA fragments that are specific for each of the *bigL* genes, sequences that encode for C-terminal regions of the BigL gene products were selected: BigL1.2078p, tta-cgg-cta-cag-gta-ttt-tta-cg and BigL1.2691p att-gga-aga-ttt-cca-agt-aac-c, BigL2.5121p tat-cta-cgc-tgc-aaa-tgg and BigL2.5865p ttg-ttg-gcg-ata-cgt-ccg, BigL3.5071p cat-aac-tct-cct-cat-aac-a and BigL3.5548p tat-gta-gag-ata-aga-tcc.

The UV Crosslinked membrane was subject to prehybridization at 42°C for 1 hour in Dig Easy Hybridization solution (Roche). Prior to hybridization, the Dig labeled probes were boiled for 10 minutes and rapidly transferred to ice for 5 minutes. The denatured probes were mixed with hybridization solution and incubated overnight with the membrane at 42°C. Following hybridization, the membranes were washed twice for 5 minute at room temperature with 2xSSC (NaCl, Sodium Citrate), 0.1%SDS. The membranes were then washed twice for 30 minutes at 42°C with 0.1 SSC, 0.1% SDS. Membranes were exposed for 1-3 minutes to Biomax ML film (Eastman Kodak, Rochester, N.Y.) for the detection of chemiluminescent products

FIGS. 1A and B shows the results of the Southern blots. Probes corresponding to DNA sequences that encode BiGL repeats hybridized to multiple DNA fragments in *L. kirschneri* and *interrogans* (FIG. 1A). In contrast, hybridization was not identified with digested genomic DNA from the non-pathogenic *L*. *biflexi.* Probes based on sequences that encode for specific C-terminal regions for each of the *L. interrogans bigL* gene products hybridized to one unique fragment in digested *L. interrogans* genomic DNA, therefore confirming that there are one copy of each of the three bigL gene identified in Example 1 (FIGS. 1B). These results illustrate a method of identifying specifically pathogenic *Leptospira* based on detection of DNA fragments not found in non-pathogenic *Leptospira.*

### Example 2B: PCR Detection of bigL gene sequences in Leptospira genomic DNA

This example illustrates the distribution of bigL gene in pathogenic *Leptospira.* In order to detect bigL genes in other *Leptospira* species, degenerate primers were designed based on an alignment for bigL genes from *L. kirschneri* strain RM52 and *L. interrogans* strain Fiocruz L1-130, identified in Example 1. The sequence of the "upstream" primer, designated BigL-lup, is 5'-(GC)AAAGTTG(TC)(AG)(TC)G(TG)CTTGGCC-3' corresponding to positions 46-65 in *bigL1* and *bigL3* (SEQ ID NO: 1 and 5), relative to A of start códon. The sequence of the "downstream" primer, designated BigL-2dn, is 5'-(GC) (AT) ACC (AG) TC (CT) GAAAA (AG) AT (AT) CC-3' corresponding to positions 506-487 in *bigL1* and *bigL3* (SEQ ID NO: 1 and 5), relative to A of the start codon. Each primer is 20 nucleotides long. These primers were designed to anneal to *bigL2* at positions 97-116 and 590-571relative to the A in bigL2's start codon (SEQ ID NO: 3).

PCR reactions were performed with purified genomic DNA from high and low-passage strains of *Leptospira.* In FIG 3., amplified DNA fragments were identified in PCR reactions with genomic DNA of strains in all four pathogenic species evaluated. Fragments had the predicted electrophoretic mobility based on the sequences of *bigL1*/*bigL3* (461 bp) and *bigL2* (494 bp). Amplified DNA fragments were not identified in the two non-pathogenic Leptospira species evaluated. Therefore this example illustrates the application of this PCR method for identifying specifically DNA from pathogenic Leptospira in samples.

### Example 2C: Reverse transcriptase-Polymerase Chain Reaction (RT-PCR) Detection of Leptospira bigL RNA

This example illustrates the detection of bigL RNA in samples. *L. kirschneri* strain RM52 was grown to late exponential phase, and total RNA was extracted from 1 x 10¹⁰ leptospiral cells using the hot-phenol method and resuspended in water following ethanol precipitation (ref). ~2 □g of leptospiral RNA was digested with 6 units of DNase I (Ambion) in 70 □l DNase I buffer (10 mM Tris-HCl pH 7.5, 25 mM MgCl₂, 1 mM CaCl₂ in 1x RNA secure from Ambion) for 30 min at 37□. To inactivate DNase I, 1.75 □l of 25 mM EDTA was added to terminate the reaction, and the enzyme was heat killed for 5 min at 70□. RT-PCR was performed using -200 ng leptospiral RNA and Omniscript RT as described (Qiagen). The following primers were used to prime the reverse transcriptase reaction:
*bigL1,* 5'-CGCAGAAATTTTAGAGGAACCTACAG-3'
*bigL2,* 5'-TTTGACTCCAAGACGCAGAGGATGAT-3'
*bigL3,* 5'-ATTTTCAAGATTTGTTCTCCAGATTT-3';
*lipL45,* 5'-ATTACTTCTTGAACATCTGCTTGAT-3'.

The RT reactions were subjected to DNA PCR using Taq polymerase (Qiagen). Prior to PCR, the following primers were added to the reactions:
*bigL1,* 5'-CTGCTACGCTTGTTGACATAGAAGTA-3'
*bigL2,* 5'-TAGAACCAACACGAAATGGCACAACA-3'
*bigL3,* 5'-ATCCGAAGTGGCATAACTCTCCTCAT-3'
*lipL45,* 5'-TGAAAAGAACATTACCAGCGTTGTA-3'.

Along with the primers added for reverse transcription, PCR products of 500 bp, 479 bp, 440 bp, and 438 bp are expected. To perform PCR, the reaction mixtures were placed in a Techne Progene thermocycler. An initial denaturation step of 95□ for 1 min was followed by 30 cycles of denaturation at 95□ for 30 sec, annealing at 53□ for 30 sec, and extension at 72□ for 30 sec. A final 72□ incubation for 30 sec was then performed.

The results in FIG. 4 show that RT-PCR method can detect BigL3 transcripts and the control LipL46 transcripts. BigL1 and BigL2 transcripts were not identified indicating that that whereas BigL3 is expressed in Leptospira, BigL1 and BigL2 may not be. Furthermore, these results demonstrate the application of the RT-PCR method to identify specific BigL gene transcripts in samples.

### EXAMPLE 3

### Expression and Purification of Recombinant BigL Proteins

This example illustrates the use of the DNA sequences of bigL genes to express and purify recombinant BigL polypeptides. Two pairs of oligonucleotides were designed for use in expressing two regions of *L. interrogans* BigL3. The first region was a region within BigL3 corresponding to the 2nd to 6th repetitive domains and corresponded to positions 131-649 of SEQ ID NO: 6 in the *L. kirschneri* BigL3DNA sequence. Oligonucleotides were designed based upon sequence of lambda *L. interrogans* BigL3 clones identified in Example 1 and their sequence are:
45B-1 5'-ATGGGACTCGAGATTACCGTTACACCAGCCATT-3'
45B-2 5'-ATTCCATGGTTATCCTGGAGTGAGTGTATTTGT-3'

PCR amplification with oligonucleotides 45B-1 and 45B-2 and purified *L. interrogans* genomic DNA was performed to obtain DNA fragments. These fragments were digested with XhoI and NcoI Enzymes (New Biolabs) and then ligated into the pRSETA expression vector (Invitrogen) (16). The cloned product was sequenced using vector specific primers and primer walking and the sequence of the 1557 bp product is shown in SEQ ID NO: 7. The predicted sequence of the encoded 519 amino acid polypeptide, designated BigL3 region 1, is shown in SEQ ID NO: 8.

A second region was selected for expression that contained the final 200 amino acids of the C-terminal region of *L. interrogans* BigL3. This region corresponded amino acid positions 1687-1886 of SEQ ID NO: 6 in *L. kirschneri* BigL3. The oligonucleotides used to clone this region are:
BIGLCTERM1 5' aac-ctc-gag-cat-aac-tct-cct-cat-aac 3'
BIGLCTERM2 5' ttc-gaa-ttc-tta-ttg-att-ctg-ttg-tct-g 3'

PCR amplification with oligonucleotides BIGLCTERM1 and BIGLCTERM2 and purified *L. interrogans* genomic DNA was performed to obtain DNA fragments. These fragments were digested with XhoI and EcoRI enzymes (New Biolabs) and then were ligated into the pRSETA expression vector (Invitrogen) (16). The cloned product was sequenced using vector specific primers and primer walking and the nucleotide sequence of the 600 bp product is shown in SEQ ID NO: 9. The predicted sequence of the encoded 200 amino acid polypeptide, designated BigL3 region 2, is shown in SEQ ID NO: 10.

Recombinant proteins, rBigL regions 1 and 2, were expressed in BL21(DE3) pLysogen (Invitrogen). Isopropyl-β-D-thiogalactopyranoside (IPTG; 2mM final concentration, Life Technologies) was added to log-phase cultures of E. *coli* BLR(DE3)pLysS (Novagen) transformed with pRSET plasmids encoding leptospiral DNA fragments for expression of His6-fusion proteins. 6M guanidine hydrochloride was used to solubilize culture pellets and His6-fusion proteins were purified by affinity chromatography with Ni2+-nitrilotriacetic acid-agarose (Qiagen and Pharmacia). The purity of eluted His6 fusion proteins was assessed by gel electrophoresis and staining with Coomassie brilliant blue. Proteins were dialyzed against PBS, 10% (v/v) glycerol, 0.025% (w/v) sodium azide. After dialysis, the protein concentration was determined with bicinchoninic acid (42). A Ponceau-S (Sigma Chem Co)-stained nitrocellulose membrane after transfer of purified BigL3 region 1 is shown in FIG 7. The relative mobility of the purified BigL3 was similar to the estimated molecular mass of approximately 58 kD, which was calculated based on the predicted amino acid sequence of the recombinant protein.

### EXAMPLE 4

### Example 4A: Detection of Antibodies against Recombinant BigL Proteins

This example illustrates two among several methods that utilize BigL polypeptides to detect antibodies in subject samples. Furthermore, this example provides methods for a serodiagnostic kits for identifying infection in subjects suspected of harboring infection.

### Immunoblot Detecting of Antibodies to BigL Polypeptides in Samples from Infected Subjects

Purified recombinant BigL3 region 2 polypeptide (lmcg/lane) (Example 3) was subjected to sodium dodecylsulfate-polyacrylamide 12% gel eloectorphoresis (SDS-PAGE) using a discontinuous buffer system and transferred to nitrocelulose membranes (Osmomics), as previously described (17). The nitrocellulose filter was blocked with TBST with 5% skimmed milk, incubated for more than 1 hour with pooled sera from patients with labortory confirmed leptospirosis, captured rat (*Rattus norvegicus*) reservoirs of *Leptosprira* which had urine and kidney cultures positive for pathogenic Leptospira, and experimental laboratory rats and rabbits, immunized with whole *L. interrogans* serovar copenhageni strain Fiocruz L1-130 lysates. As control experiments, incubations were performed with sera from health individuals from Brazil, captured rats who had no culture or serologic evidence for a *Leptospira* infection and laboratory rats and rabbits prior to immunization. Sera were diluted 1:100 prior to use. After washing, membranes were incubated with goat anti-human gamma chain antibody conjugated to alkaline phosphatase (Sigma), diluted 1:1000, for more than 1 hour. Antigen-antibody complexes were detected by color reaction through with NBT (0,3 mg/ml) and BCIP (0,15 mg/ml). Pooled sera from leptospirosis patients, captured rats who were infected with pathogenic leptospires strongly recognized purified recombinant BigL 3 region 1 protein. However, rats immunized with whole Leptospira lysates did not visibly bind to the BigL3 polypeptide, indicating that although BigL3 is expressed in cultured leptospires (Example 2, FIG. 4), there may be differential expression of the bigL3 gene. Sufficient quantities of native BigL3 protein may not be present in vitro whereas, during natural infection, leptospires in vivo produce sufficient quantities of BigL3 to induce a strong immune response. Furthermore, this example illustrates that a spectrum of animals produce an immune response to BigL3 during infection and detection of this immune response, and detection of antibodies to recombinant BigL3 polypeptide can be used as a method to identify infection in subjects.

To further illustrate the use of a detection method for antibodies against recombinant BigL3 polypeptide, an immunoblot evaluation was performed with individual sera of patients with laboratory-confirmed leptospirosis, healthy individuals from Brazil and US and patients hospitalized or evaluated in ambulatory clinics with diagnoses other than leptospirosis. The microagglutination test and culture isolation was used to confirm the diagnosis of leptospirosis in patients with clinically-suspected disease (5). The collection of sera from leptospirosis patients was during five-year surveillance for leptospirosis in the city of Salvador, Brazil. The collection of sera from control individuals was obtained from pre-existing serum banks of hospitalized and clinic patients and healthy individuals from Salvador, Brazil and through donations from the Center for Disease Control and Prevention, USA. A list of the sera used is shown in TABLE 1. Sera diluted 1:100 were analyzed following the method described above. The finding of any visible colorization of the 1mcg band of recombinant BigL3 region 1 polypeptide in the immunoblot was considered a positive reaction.

FIG. 8 illustrates that sera from individual leptospirosis patients react with recombinant BigL3. Table 1 summarizes the findings that demonstrate that more than 90% of hospitalized patients and approximately 70% of outpatients with leptospirosis react to rBigL3 during active infection. All (100%) of the leptospirosis patients react to rBigL3 during the convalescent-phase of their illness. Table 2 compares seroreactivity to rBigL3 with standard diagnostic tests. RBigL3 seroreactivity was greater during the initial phase of illness to those observed for standard diagnostic tests. Healthy individuals from the US and 88% of the healthy individuals from Brazil do not react to rBigL3, demonstrating that this reaction to rBigL3 is specific. The specificity of the reaction increases to 100% when it is calculated based on the frequency of IgM seroreactivity among healthy Brazilian individuals. Together, these finding illustrate that the method has utility as a serological marker of active infection and is the basis for a kit that can be used for diagnosis with leptospirosis.

Table 1 also summarizes findings for rBigL3 seroreactivity in endemic regions that have high risk for leptospirosis. 25% of the population that resides in these regions demonstrate rBigL3 IgG seropositivity, indicating that this reaction may be a useful marker to identify past infection. Among patients with confirmed leptospirosis, 56% were seroreactive agains rBigL3 during the period two years after their infection with leptospirosis (Table 2). In the period between 2 and 4 years after infection with leptospirosis, 18% demonstrated rBigL3 seroreactivity. Together, these findings illustrate that a kit based on the immunoblot method can detect a past infection with leptospirosis.

### Example 4B: ELISA-based Detection of Antibodies to BigL Polypeptides in Samples from Infected Subjects

This example illustrates that ELISA methods are useful in detecting antibodies to BigL polypeptides and in identifying patients with leptospirosis among those with suspected infection. Flat-bottomed polystyrene microtiter plates (Corning) were coated at 4° C overnight with His₆-fusion rBigL3, 0.5 - 100 ng/well, suspended in 0.05 M sodium carbonate, pH 9.6 (16). The plates were washed twice with distilled water and three times with PBS, 0.05% (v/v) Tween 20 (PBST). Plates were incubated with blocking solution (PBST/1% [w/v] bovine serum albumin) for 2 hours at room temperature and after four washes with PBST, were stored at -20°C until use. Wells were incubated with 50 µl of sera, diluted 50 to 200-fold in blocking solution, for 1 hour at room temperature with agitation. After four washes with PBST, wells were incubated with 50 µl of 5,000 to 20,000-fold dilutions of anti-human µ or γ-chain goat antibodies conjugated to horseradish peroxidase (Sigma) for 1 hour at room temperature with agitation. Afterwards, plates were washed twice with PBST and three times with PBS and incubated with 50 ul/well of 0.01% (w/v) 3,3',5,5'-tetramethylbenzidine in substrate buffer (0.03% [v/v] hydrogen peroxide, 25 mM citric acid, 50 mM Na₂HPO₄, pH 5.0) for 20 minutes in the dark at room temperature. The color reaction was stopped by adding 25 uL 2 N H₂SO₄ and the absorbance at 450 nm was measured in an Emax microplate reader (Molecular Devices, Sunnyvale, CA).

Initial assays were performed to determine the antigen concentration (mcgg/well) that best discriminated between ELISA reactions of serum samples from laboratory-confirmed leptospirosis cases (*n* = 4) and healthy individuals from an endemic area for leptospirosis in Brazil (*n* = 4). Checkerboard titrations were performed with 50, 100 or 200-fold serum dilutions and antigen concentrations per well of 25, 50, 100 and 200 ng. FIG. 6 illustrates that significantly increased absorbance values were observed at all serum dilutions and rBigL3 polypeptide concentrations for leptospirosis patients than for control individuals.

In subsequent assays to determine sensitivity and specificity, plates were coated with 50ng of rBigL3. Incubations were performed with 50 and 10,000-fold dilutions of primary sera and secondary antibody conjugate, respectively. Individual serum samples were tested in duplicate and the means of the two measurements were calculated for analysis. Paired measurements that differed by greater than 10% were retested. One positive control serum sample which reacted with all recombinant antigens and one negative control serum sample were included, in duplicate, on each plate as a quality control measure. FIG. 7 illustrates that leptospirosis patients in the acute phase of illness had significantly increased absorbances than control individuals for IgM and IgG seroreactivity (FIG. 7). These differences increased when comparing absorbance values for patients in their convalescent-phase of illness. These experiments illustrate that an ELISA-based method for detecting antibodies against rBigL3 polypeptide is useful for identifying infection with leptospirosis and can be used as a kit for diagnosis.

### EXAMPLE 5

### Induction of an Immune Response against Leptospira in Subjects

This example illustrates that an immune response against BigL proteins can be induced via immunization with recombinant BigL proteins. Purified recombinant BigL3 polypeptide derived from *L. interrogans* was obtained with the method described in Example 3. Laboratory rats (Wistar strain) were immunized with 40 mcgs of rBigL3 in Freund's adjuvant (Sigma), and inoculated subcutaneously. Additional immunizations were performed with 20 mcgs of rBigL3 at weeks 3 and 6. Blood was collected 7 weeks after primary immunization and process for serum. Immunoblots with rBigL3 (1mcg/lane) were prepared as in Example 4. FIG. 9 illustrates the seroreactivity of rBigL3-immunized rats. rBigL3 was an effective immunogen inducing immunoblot rBigL3 seroreactivity with titers of greater than 1:2500 after a total of three immunizations. Furthermore, antibodies raised to rBigL3 polypeptide recognized native antigens in whole *Leptospira* lysates (10⁸ leptospires per lane) (FIG. 9). A band with relative mobility at 200kD is faintly stained in immunoblots as are more intensely staining bands with lower relative mobility, which may represent degradation of the 200kD or high molecular weight BigL proteins. Seroreactivity against these native antigens is specific since no reactions are observed in the pre-immune sera.

Immunogenicity experiments were performed with purified recombinant BigL polypeptides derived L. kirschneri. Purified recombinant proteins were loaded onto a preparative 12% SDS-PAGE gel and allowed to migrate into the separating gel by electrophoresis. A band containing 100-200smcg of recombinant protein was excised from the gel, desiccated, ground to powder, dissolved in 1 ml of water, mixed with 1 ml complete Freund's adjuvant (Sigma), and inoculated subcutaneously and intramuscularly in New Zealand white rabbits (Harlan Sprague Dawley) that were free of leptospiral antibodies. Additional immunizations with similar amounts of fusion protein in powdered acrylamide gel mixed with incomplete Freund's adjuvant (Sigma) were administered at four and eight weeks after primary immunization. Blood was collected from the rabbits ten weeks after primary immunization and processed for serum (Harlow, 1988). Immunoblots were performed as previously described (Guerreiro et al Infect Immun 2001) with concentrations of 108 leptospires per lane.

FIG 10. illustrates that immunization with rBigL3 derived from *L. kirschneri* induces high level antibody titers to native BigL3 polypeptides in *L. kirschneri* and other pathogenic Leptospira species such as *L. interrogans.* Together these findings illustrate that immunization with rBigL polypeptides induces an immune response against species of pathogenic spirochetes other than the species used to design the recombinant rBigL polypeptide. Furthermore, the antibodies produced by this method of immunization can be used to detect pathogenic spirochetes in samples.

Finally, this example demonstrates that the presence of native BigL polypeptides is observed in virulent low culture passaged strains and not in avirulent attenuated high culture passaged strains (Fig 10). Sera from rBigL3-immunized rabbits recognized a predicted 200kDa corresponding to BigL3 in whole *Leptospira* lysates of virulent and not avirulent attenuated strains. This example illustrates that BigL proteins are markers for virulence and that antibodies against BigL proteins can be used as a method to identify virultent strains. Since BigL may be itself a virulence factor, induction of an immune response to BigL proteins as demonstrated in the example will be useful for application as a vaccine.

**Table 1. Detection of IgG and gM antibodies against rBigL and rLipL32 in sera from leptospirosis patients and control groups as determined by the Western Blot method.**

| | No. tested | rBigL3 seroreactivity | | | rLipL32 seroreactivity | | |
|---|---|---|---|---|---|---|---|
| Study group | | IgM | IgG | IgM or IgG | IgM | IgG | IgM or IgG |
| | No. positive reactions (%) | | | | | | |
| Hospitalized cases of confirmed leptospirosis | | | | | | | |
| Acute-phase | 52 | 37 (71) | 46 (88) | 48 (92) | 22 (42) | 21 (50) | 38 (73) |
| Convalescent-phase | 52 | 19 (37) | 52 (100) | 52 (100) | 21 (40) | 45 (86) | 46 (88) |

| Outpatient cases of confirmed leptospirosis | | | | | | | |
|---|---|---|---|---|---|---|---|
| Acute-phase | 14 | 6 (42) | 8 (57) | 9 (64) | 2 (14) | 2 (14) | 3 (21) |
| Convalescent-phase | 14 | 7 (50) | 14 (100) | 14(100) | 6 (42) | 5 (36) | 8 (57) |

| Healthy individual control groups | | | | | | | |
|---|---|---|---|---|---|---|---|
| Non-endemic area (USA) | 30 | 0 (0) | 0 (0) | 0 (0) | 0 (0) | 0 (0) | 0 (0) |
| Endemic area (Brazil) | 40 | 0 (0) | 5 (12) | 5 (12) | 2 (6) | 0 (0) | 2 (6) |
| High risk endemic area (Brazil) | 40 | 0 (0) | 10 (25) | 10 (25) | 4 (10) | 5 (12) | 8 (20) |

| Patient control groups | | | | | | | |
|---|---|---|---|---|---|---|---|
| Dengue | 15 | 0 (0) | 0 (0) | 0 (0) | 0 (0) | 0 (0) | 0 (0) |
| Lyme disease | 15 | 0 (0) | 0 (0) | 0 (0) | 0 (0) | 0 (0) | 0 (0) |
| VDRL-positive | 20 | 0 (0) | 1 (5) | 1 (5) | 0 (0) | 1 (5) | 1 (5) |

**Table 2. Comparison of the rBigL3and rLipL32-based Western blot with standard diagnostic tests for leptospirosis.**

| Time period after initiation of illness | No. tested | Standard diagnostic evaluation | | | rBigL Western blot seroreactivity | | | rLipL32 Western blot seroreactivity | | |
|---|---|---|---|---|---|---|---|---|---|---|
| | | Median maximum reciprocal MAT titer (range) | Reciprocal MAT titer □ 100 | ELISA-IgM | IgM | IgG | IgM or IgG | IgM | IgG | IgM or IgG |
| | | | No. positive reactions (%) | | | | | | | |
| Acute phase (N = 52)*^{a}* | | | | | | | | | | |
| 2-6 days | 21 | 200 (0-1600) | 12 (57) | 11 (52) | 12 (57) | 16 (76) | 17 (81) | 8 (38) | 8 (38) | 12 (57) |
| 7-15 days | 31 | 400 (0-3200) | 17 (55) | 20 (91) | 25 (81) | 30 (97) | 31 (100) | 14 (45) | 23 (74) | 26 (84) |

| Early convalescent phase (N = 52) | | | | | | | | | | |
|---|---|---|---|---|---|---|---|---|---|---|
| 16-21 days | 21 | 800 (200-12800) | 21 (100) | 15 (100) | 7 (33) | 21 (100) | 21 (100) | 8 (38) | 18 (86) | 19 (90) |
| 21-30 days | 31 | 1600 (0-6400) | 31 (100) | 21 (100) | 12 (39) | 31 (100) | 31 (100) | 13 (42) | 27 (87) | 27 (87) |

| Late convalescent phase (N = 59) | | | | | | | | | | |
|---|---|---|---|---|---|---|---|---|---|---|
| 0-23 months | 25 | 400 (0-800) | 21 (84) | 24 (96) | 0 (0) | 14 (56) | 14 (56) | 2 (8) | 2 (8) | 3 (12) |
| 24-47 months | 17 | 400 (100-1600) | 17 (100) | 7 (41) | 0 (0) | 3 (18) | 3 (18) | 2 (12) | 2 (12) | 3 (18) |
| 48-78 months | 17 | 200 (0-800) | 15 (88) | 5 (29) | 0 (0) | 3 (18) | 3 (18) | 2 (12) | 1 (6) | 3 (18) |

| | | | | | | | | | | |
|---|---|---|---|---|---|---|---|---|---|---|
| *^{a}* Acute-phase serum samples were collected upon hospital admission. | | | | | | | | | | |

Accordingly, the invention is limited only by the following claims.

### References

1. Levett PN. Leptospirosis. Clin Microbiol Rev. 2001; 14(2):296-326.
2. Faine SB, Adler B, Bolin C, Perolat P. Leptospira and leptospirosis. 2nd ed Melbourne, Australia: MediSci; 1999.
3. Farr RW. Leptospirosis. Clin Infect Dis. 1995;21(1):1-6; quiz 7-8.
4. Lomar AV, Diament D, Torres JR. Leptospirosis in Latin America. Infect Dis Clin North Am. 2000; 14(1):23-39, vii-viii.
5. Ko AI, Galvao Reis M, Ribeiro Dourado CM, Johnson WD, Jr., Riley LW. Urban epidemic of severe leptospirosis in Brazil. Salvador Leptospirosis Study Group. Lancet. 1999; 359 (9181):820-5.
6. Bughio NI, Lin M, Surujballi OP. Use of recombinant flagellin protein as a tracer antigen in a fluorescence polarization assay for diagnosis of leptospirosis. Clin Diagn Lab Immunol. 1999; 6(4):599-605.
7. Park SH, Ahn BY, Kim MJ. Expression and immunologic characterization of recombinant heat shock protein 58 of Leptospira species: a major target antigen of the humoral immune response. DNA Cell Biol. 1999; 18(12):903-10.
**8.** Haake DA, Walker EM, Blanco DR, Bolin CA, Miller MN, Lovett MA. Changes in the surface of Leptospira interrogans serovar grippotyphosa during in vitro cultivation. Infect Immun. 1991; 59(3):1131-40.
**9.** Haake DA, Champion CI, Martinich C, et al. Molecular cloning and sequence analysis of the gene encoding OmpL1, a transmembrane outer membrane protein of pathogenic Leptospira spp. J Bacteriol. 1993; 175(13):4225-34.
**10.** Haake DA, Martinich C, Summers TA, et al. Characterization of leptospiral outer membrane lipoprotein LipL36: downregulation associated with late-log-phase growth and mammalian infection. Infect Immun. 1998; 66(4):1579-87.
11. Haake DA, Mazel MK, McCoy AM, et al. Leptospiral outer membrane proteins OmpL1 and LipL41 exhibit synergistic immunoprotection. Infect Immun. 1999; 67(12):6572-82.
12. Haake DA, Chao G, Zuerner RL, et al. The leptospiral major outer membrane protein LipL32 is a lipoprotein expressed during mammalian infection. Infect Immun. 2000; 68(4):2276-85.
13. Shang ES, Exner MM, Summers TA, et al. The rare outer membrane protein, OmpL1, of pathogenic Leptospira species is a heat-modifiable porin. Infect Immun. 1995; 63(8):3174-81.
14. Shang ES, Summers TA, Haake DA. Molecular cloning and sequence analysis of the gene encoding LipL41, a surface-exposed lipoprotein of pathogenic Leptospira species. Infect Immun. 1996; 64(6):2322-30.
15. Yelton DB, Charon NW. Cloning of a gene required for tryptophan biosynthesis from Leptospira biflexa serovar patoc into Escherichia coli. Gene. 1984; 28(2):147-52.
16. Flannery B, Costa D, Carvalho FP, et al. Evaluation of recombinant Leptospira antigen-based enzyme-linked immunosorbent assays for the serodiagnosis of leptospirosis. Journal of Clinical Microbiology. 2001; 39(9):3303-3310.
17. Guerreiro H, Croda J, Flannery B, et al. Leptospiral proteins recognized during the humoral immune response to leptospirosis in humans. Infect Immun. 2001; 69(8):4958-68.

### SEQUENCE LISTINGS

1) GENERAL INFORMATION
   I.a) APPLICANTS: FIOCRUZ and KO, Albert I.; HAAKE, David A.; REIS, Mitermayer Galvão; MATSUNAGA, James; CRODA, Julio Henrique Rosa; SIQUEIRA, Isadora Cristina; RILEY, Lee W.; BAROCCHI, Michele; YOUNG, Tracy Ann.
   I.b) ADDRESS:
      Centro de Pesquisas Gonçalo Moniz
      Fundaça̅o Oswaldo Cruz/MS
      Rua Waldemar Falcão, 121
      Salvador, Bahia 40295-001
      Brazil
   II) TITLE OF INVENTION: "Protein family with repetitive Bacterial-Ig-like (Big) domains present in *Leptospira* species"
   III) NUMBER OF SEQUENCES: 10 (ten).
   IV) COMPUTER READABLE FORM:
   IV.a) MEDIUM TYPE: Floppy disk
   IV.b) COMPUTER: IBM PC compatible
   IV.c) OPERATING SYSTEM: PC-DOS/MS-DOS.
2) GENERAL INFORMATION FOR SEQUENCES:
   I.a) Identifier Number for Sequence: SEQ ID NO: 1
   II) Sequence Characteristics:
   II.a) LENGTH: 3672 base pairs
   II.b) TYPE: DNA
   II.c) STRANDEDNESS: single strand
   II.d) TOPOLOGY: linear
   III) Position in the genome:
   III.a) organism: *Leptospira kirschneri*
   III.b) Name/key: CDS
   III.c) position in the map:(0) - (3672)

   I.b) Identifier Number of Sequence: SEQ ID NO2
   II) Sequence Characteristics:
   II.a) LENGHT: 1224
   II.b) TYPE: amino acid
   II.c) TOPOLOGY: linear
   III) TYPE: peptide
   IV) SOURCE: N-terminal to C-terminal portion

   I.c) Identifier Number of Sequence: SEQ ID NO: 3
   II) Sequence Characteristics:
   II.a) LENGTH: 5863 base pairs
   II.b) TYPE: nucleic acid
   II.c) STRANDEDNESS: single strand
   II.d) TOPOLOGY: linear
   III) Position in the genome:
   III.a) organism: *Leptospira kirschneri*
   III.b) Name/key: CDS
   III.c) position in the map:(0) - (5863)

   I.d) Identifier Number of the Sequence: SEQ ID NO 4
   II) Sequence Characteristic:
   II.a) LENGHT: 1954
   II.b) TYPE: amino acid
   II.c) TOPOLOGY: linear
   III) MOLECULE TYPE: peptide
   IV) SOURCE: N-terminal to C-terminal portion

   I.e) Identifier Number of the Sequence: SEQ ID NO: 5
   II) Sequence Characteristics:
   II.a) LENGTH: 5658 base pairs
   II-b) TYPE: nucleic acid
   II.c) STRANDEDNESS: single strand
   II.d) TOPOLOGY: linear
   III) Position in the genome:
   III.a) organism: *Leptospira kirschneri*
   III.b) Name/key: CDS
   III.c) Position in the map:(0) - (5658)

   I.f) Identifier Number of the Sequence: SEQ ID NO: 6
   II) Sequence Characteristic:
   II.a) LENGHT: 1886
   II.b) TYPE: amino acid
   II.c) TOPOLOGY: linear
   III) MOLECULE TYPE: peptide
   IV) SOURCE: N-terminal to C-terminal portion

   I.g) Identifier Number of the Sequence: SEQ ID NO: 7
   II) Sequence Characteristic:
   II.a) LENGTH: 1557 base pairs
   II.b) TYPE: nucleic acid
   II.c) STRANDEDNESS: single strand
   II.d) TOPOLOGY: linear
   III) Position in the genome:
   III.a) organism: *Leptospira interrogans*
   III.b) Name/key: CDS
   III.c) Position in the map: (0) - (1557)

   I.h) Identifier Number of the Sequence: ID SEQ NO: 8
   II) Sequence Characteristic:
   II.a) LENGTH: 519
   II.b) TYPE: amino acid
   II.c) TOPOLOGY: linear
   III) MOLECULE TYPE: peptide
   IV) SOURCE: N-terminal to C-terminal portion (0) - (519)

   I.i) Identifier Number of the Sequence: SEQ ID NO: 9
   II) Sequence Characteristic:
   II.a) LENGTH: 600 base pairs
   II.b) TYPE: nucleic acid
   II.c) STRANDEDNESS: single strand

   II.d) TOPOLOGY: linear
   III) Position in the genome:
   III.a) organism: *Leptospira interrogans*
   III.b) Name/key: CDS
   III.c) Position in the map: (0) - (600)

   I.j) Identifier Number of the Sequence: SEQ ID NO: 10
   II) Sequence Characteristic:
   II.a) LENGTH: 200
   II.b) TYPE: amino acid
   II.c) TOPOLOGY: linear
   III) MOLECULE TYPE: peptide
   IV) SOURCE: N-terminal to C-terminal portion (0) - (200)

## Claims

1. A substantially purified polypeptide having an amino acid sequence as set forth in SEQ ID NO:2.

2. An isolated polynucleotide segment having a sequence selected from the group consisting of
(a) a polynucleotide sequence encoding an amino acid sequence as set forth in SEQ ID NO:2; and
(b) a polynucleotide sequence SEQ ID NO: 1.

3. A substantially purified polypeptide having an amino acid sequence as set forth in SEQ ID N0:4.

4. An isolated polynucleotide having a sequence selected from the group consisting of:
(a) a polynucleotide sequence encoding an amino acid sequence as set forth in SEQ ID NO:4; and
(b) a polynucleotide sequence SEQ ID NO:3.

5. A substantially purified polypeptide having an amino acid sequence as set forth in SEQ ID NO:6.

6. An isolated polynucleotide having a sequence selected from the group consisting of:
(a) a polynucleotide sequence encoding an amino acid sequence as set forth in SEQ ID NO:6; and
(b) a polynucleotide sequence SEQ ID NO:5.

7. A substantially purified polypeptide sequence selected from SEQ ID NO: 8.

8. An isolated polynucleotide segment having a sequence selected from the group consisting of:
(a) a polynucleotide sequence encoding an amino acid sequence as set forth in SEQ ID NO: 8; and
(b) a polynucleotide SEQ ID NO:7.

9. A substantially purified polypeptide sequence selected from SEQ ID NO: 10.

10. An isolated polynucleotide segment having a sequence selected from the group consisting of:
(a) a polynucleotide sequence encoding an amino acid sequence as set forth in SEQ ID NO: 10;
(b) a polynucleotide sequence SEQ ID NO: 9.

11. Antibodies that bind specifically to the substantially purified polypeptide defined in claims 1, 3, 5, 7 or 9.

12. A method for detecting pathogens in a sample which comprises contacting a sample suspected of containing a pathogenic spirochete with a reagent that specifically binds to the pathogen-specific cell component and detecting binding of the reagent to the component, wherein the component is selected from SED ID NOs 2, 4, 6, 8 and 10.

13. The method according to claim 12 wherein the reagent that binds to the pathogen-specific cell component is an antibody against the BigL1, BigL2 or BigL3 polypeptide or polypeptides of SEQ ID NOs 2, 4, 6, 8, 10.

14. A kit useful for the detection of BigL1, BigL2, and BigL3 polypeptides; or antibodies that bind to BigL1, BigL2, BigL3 polypeptides or polypeptides, wherein the proteins present in the kit are selected from SEQ ID Nos 2, 4, 6, 8 or 10 or antibodies specifically binding to said proteins.

## Patentansprüche

1. Im Wesentlichen gereinigtes Polypeptid, das eine Aminosäuresequenz hat, wie sie in SEQ ID NO: 2 angegeben ist.

2. Isoliertes Polynucleotid-Segment, das eine Sequenz hat, die ausgewählt ist aus der Gruppe, bestehend aus:
(a) einer Polynucleotidsequenz, die für eine Aminosäuresequenz codiert, wie sie in SEQ ID NO: 2 angegeben ist, und
(b) einer Polynucleotidsequenz SEQ ID NO: 1.

3. Im Wesentlichen gereinigtes Polypeptid, das eine Aminosäuresequenz hat, wie sie in SEQ ID NO: 4 angegeben ist.

4. Isoliertes Polynucleotid, das eine Sequenz hat, die ausgewählt ist aus der Gruppe, bestehend aus:
(a) einer Polynucleotidsequenz, die für eine Aminosäuresequenz codiert, wie sie in SEQ ID NO: 4 angegeben ist, und
(b) einer Polynucleotidsequenz SEQ ID NO: 3.

5. Im Wesentlichen gereinigtes Polypeptid, das eine Aminosäuresequenz hat, wie sie in SEQ ID NO: 6 angegeben ist.

6. Isoliertes Polynucleotid, das eine Sequenz hat, die ausgewählt ist aus der Gruppe, bestehend aus:
(a) einer Polynucleotidsequenz, die für eine Aminosäuresequenz codiert, wie sie in SEQ ID NO: 6 angegeben ist, und
(b) einer Polynucleotidsequenz SEQ ID NO: 5.

7. Im Wesentlichen gereinigte Polypeptidsequenz, ausgewählt aus SEQ ID NO: 8.

8. Isoliertes Polynucleotid-Segment, das eine Sequenz hat, die ausgewählt ist aus der Gruppe, bestehend aus:
(a) einer Polynucleotidsequenz, die für eine Aminosäuresequenz codiert, wie sie in SEQ ID NO: 8 angegeben ist, und
(b) einem Polynucleotid SEQ ID NO: 7.

9. Im Wesentlichen gereinigte Polypeptidsequenz, ausgewählt aus SEQ ID NO: 10.

10. Isoliertes Polynucleotid-Segment, das eine Sequenz hat, ausgewählt aus der Gruppe, bestehend aus:
(a) einer Polynucleotidsequenz, die für eine Aminosäuresequenz, wie sie in SEQ ID NO: 10 angegeben ist, codiert;
(b) einer Polynucleotidsequenz SEQ ID NO: 9.

11. Antikörper, die spezifisch an das im Wesentlichen gereinigte Polypeptid, das in den Ansprüchen 1, 3, 5, 7 oder 9 definiert ist, binden.

12. Verfahren zum Detektieren von Pathogenen in einer Probe, umfassend In-Kontakt-Bringen einer Probe, von der angenommen wird, dass sie eine pathogene Spirochäte enthält, mit einem Reagens, das spezifisch an die Pathogen-spezifische Zellkomponente bindet, und Detektieren der Bindung des Reagens an die Komponente, wobei die Komponente ausgewählt wird aus SEQ ID NO: 2, 4, 6, 8 und 10.

13. Verfahren nach Anspruch 12, wobei das Reagens, das die Pathogen-spezifische Zellkomponente bindet, ein Antikörper gegen das BigL1-, BigL2-, oder BigL3-Polypeptid oder Polypeptide von SEQ ID NO: 2, 4, 6, 8, 10 ist.

14. Kit, der für die Detektion von BigL1-, BigL2- und BigL3-Polypeptiden oder von Antikörpern, die an BigL1-, BigL2-, BigL3-Polypeptide oder Polypeptide binden, einsetzbar ist, wobei die im Kit vorliegenden Proteine ausgewählt sind aus SEQ ID NO: 2, 4, 6, 8 oder 10, oder Antikörpern, die spezifisch an die genannten Proteine binden, einsetzbar ist.

## Revendications

1. Polypeptide sensiblement purifié ayant une séquence d'acides aminés comme présentée dans SEQ ID N° 2.

2. Segment de polynucléotide isolé ayant une séquence choisie dans le groupe consistant en :
(a) une séquence polynucléotidique codant une séquence d'acides aminés comme présentée dans SEQ ID N° 2 ; et
(b) une séquence polynucléotidique SEQ ID N° 1.

3. Polypeptide sensiblement purifié ayant une séquence d'acides aminés comme présentée dans SEQ ID N° 4.

4. Polynucléotide isolé ayant une séquence choisie dans le groupe consistant en :
(a) une séquence polynucléotidique codant une séquence d'acides aminés comme présentée dans SEQ ID N° 4 ; et
(b) une séquence polynucléotidique SEQ ID N° 3.

5. Polypeptide sensiblement purifié ayant une séquence d'acides aminés comme présentée dans SEQ ID N° 6.

6. Polynucléotide isolé ayant une séquence choisie dans le groupe consistant en :
(a) une séquence polynucléotidique codant une séquence d'acides aminés comme présentée dans SEQ ID N° 6 ; et
(b) une séquence polynucléotidique SEQ ID N° 5.

7. Séquence polypeptidique sensiblement purifiée choisie dans SEQ ID N° 8.

8. Segment de polynucléotide isolé ayant une séquence choisie dans le groupe consistant en :
(a) une séquence polynucléotidique codant une séquence d'acides aminés comme présentée dans SEQ ID N° 8 ; et
(b) un polynucléotide SEQ ID N° 7.

9. Séquence polypeptidique sensiblement purifiée choisie dans SEQ ID N° 10.

10. Segment de polynucléotide isolé ayant une séquence choisie dans le groupe consistant en :
(a) une séquence polynucléotidique codant une séquence d'acides aminés comme présentée dans SEQ ID N° 10 ; et
(b) une séquence polynucléotidique SEQ ID N° 9.

11. Anticorps qui se lient spécifiquement au polypeptide sensiblement purifié défini dans les revendications 1, 3, 5, 7 ou 9.

12. Procédé pour détecter des agents pathogènes dans un échantillon qui comprend les étapes consistant à mettre en contact un échantillon suspecté de contenir un spirochète pathogène avec un réactif qui se lie spécifiquement au composant cellulaire spécifique d'agent pathogène et à détecter la liaison du réactif au composant, le composant étant choisi parmi SEQ ID N° 2, 4, 6, 8 et 10.

13. Procédé selon la revendication 12 dans lequel le réactif qui se lie au composant cellulaire spécifique d'agent pathogène est un anticorps dirigé contre le polypeptide BigL1, BigL2 ou BigL3 ou les polypeptides de SEQ ID N° 2, 4, 6, 8, 10.

14. Kit utile pour la détection des polypeptides, BigL1, BigL2 et BigL3 ou anticorps qui se lient aux polypeptides BigL1, BigL2, BigL3, les protéines présentes dans le kit étant choisies parmi SEQ ID N° 2, 4, 6, 8 ou 10 ou des anticorps se liant spécifiquement aux dites protéines.
